# EUROPEAN PATENT APPLICATION

(11) **EP 1 760 078 A1**
(43) Date of publication of application: **07.03.2007**
(21) Application number: 05384007.0
(22) Date of filing: 15.07.2005
(51) Int. Cl.: C07D 231/06

(54) **Substituted pyrazoline compounds; methods for their preparation**

(71) Applicant: LABORATORIOS DEL DR. ESTEVE, S.A., 08041 Barcelona (ES)
(72) Inventor: Torrens Jover, Antonio, 08221 Terrasa (Barcelona) (ES); Yenes Minguez, Susana, 08750 Molins de rei (Barcelona) (ES)

(57) **Abstract**

Methods for obtaining the substituted pyrazoline compounds of general formula (Ia) as well as the intermediates of general formula (III) to this compound. Thus 5-(R¹⁰,R¹¹ ,R¹²-phenyl)-1-(R¹³,R¹⁴,R¹⁵-phenyl)-N-(piperidin-1-yl)-4,5-dihydro-1H-pyrazole-3-carboxamide (II) can be obtained by the reaction of an activated carbonyl compound derived from 5-(R¹⁰,R¹¹ ,R¹²-phenyl)-1-(R¹³,R¹⁴,R¹⁵-phenyl)-N-(piperidin-1-yl)-4,5-dihydro-1H-pyrazole-3-carboxylic acid (IIIa) with N-aminopiperidine in the presence or absence of an acid acceptor or suitable condensing agent.

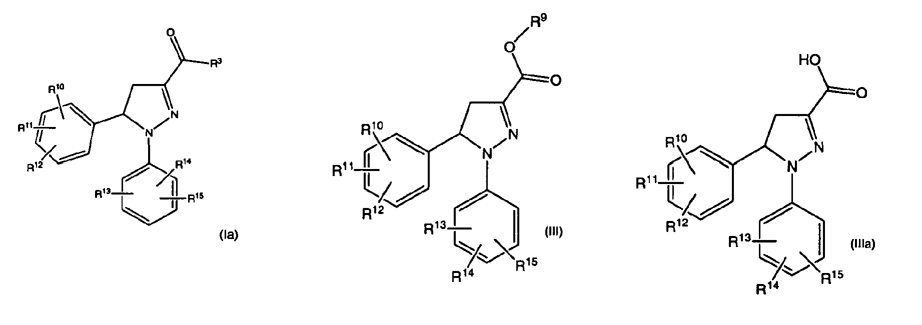

## Description

The present invention relates to methods for preparation of substituted pyrazoline compounds.

Cannabinoids are compounds, which are derived from the cannabis sativa plant which is commonly known as marijuana. The most active chemical compound of the naturally occurring cannabinoids is tetrahydrocannabinol (THC), particularly Δ⁹-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated CB₁ and CB₂ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the CB₁-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide methods for obtaining novel compounds for use as active substances in medicaments. In particular, these active substances should be suitable for the modulation of Cannabinoid receptors, more particularly for the modulation of Cannabinoid 1 (CB₁) receptors.

Said object was achieved by providing methods for obtaining the substituted pyrazoline compounds of general formula I given below, their stereoisomers, corresponding salts and corresponding solvates thereof.

It has been found that these compounds have a high affinity for cannabinoid receptors, particularly for the CB₁-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Furthermore, these pyrazoline compounds show relatively weak Herg channel affinity, thus a low risk of prolongation of the QT-interval is to be expected for these compounds.

Thus, in one of its aspects the present invention relates to different methods for obtaining substituted pyrazoline compounds of general formula I, 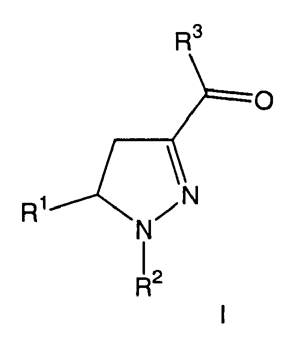 wherein

R¹ represents an optionally at least mono-substituted phenyl group,

R² represents an optionally at least mono-substituted phenyl group, R³ represents a saturated or unsaturated, optionally at least mono-substituted, optionally at least one heteroatom as ring member containing cycloaliphatic group, which may be condensed with an optionally at least mono-substituted mono- or polycyclic ring system, or R³ represents an optionally at least mono-substituted aryl or heteroaryl group, which may be condensed with an optionally at least mono-substituted mono- or polycyclic ring system, or R³ represents an -NR⁴R⁵-moiety,

R⁴ and R⁵, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R⁶-moiety, or an -NR⁷R⁸-moiety,

R⁶ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

R⁷ and R⁸, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Particularly preferably the following provisos (disclaimers) apply for the pyrazoline compounds produced of general formula I given above:
that R⁴ and R⁵ do not both represent a hydrogen atom, and
that if one of the residues R⁴ and R⁵ represents a hydrogen atom or an alkyl group, which is optionally at least mono-substituted with an alkoxy group, an alkoxyalkoxy group, a halogen atom or a phenyl group, the other one of these residues R⁴ and R⁵ does not represent a pyrid-2-yl group, which is optionally mono-substituted in the 5-position, a pyrid-5-yl group, which is optionally mono-substituted in the 2-position, a pyrimid-5-yl group, which is optionally mono-substituted in the 2-position, a pyridaz-3-yl group, which is optionally mono-substituted in the 6-position, a pyrazin-5-yl group, which is optionally mono-substituted in the 2-position, a thien-2-yl group, which is optionally mono-substituted in the 5 position, a thien-2-yl group, which is optionally at least mono-substituted in the 4-position, a benzyl group, which is optionally mono-substituted in the 4-position of the ring, a phenethyl group, which is optionally mono-substituted in the 4-position of the ring, an optionally mono-, di- or tri-substituted phenyl group, a di-substituted phenyl group, wherein the two substituents together form an ―OCH₂O-, -OCH₂CH₂O- or -CH₂CH₂O- chain, which is optionally substituted with one or more halogen atoms or one or two methyl groups, an ―NH-phenyl-moiety, wherein the phenyl group may be mono-substituted in the 4-position, and
that if one of the residues R⁴ and R⁵ represents an alkynyl group, the other one of these residues R⁴ and R⁵ does not represent a phenyl group, which is optionally substituted in the 4-position, and
that if one of the residues R⁴ and R⁵ represents a hydrogen atom or a linear or branched, saturated or unsaturated, unsubstituted or substituted aliphatic radical, the other one of these residues R⁴ and R⁵ does not represent an unsubstituted or substituted thiazole group or an unsubstituted or substituted [1,3,4]thiadiazole group.

A mono- or polycyclic ring-system according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

The term "condensed" according to the present invention means that a ring or ring-system is attached to another ring or ring-system, whereby the terms "annulated" or "annelated" are also used by those skilled in the art to designate this kind of attachment.

If one or more of the residues R³-R⁸ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched C₁₋₆-alkoxy, branched or unbranched C₁₋₆-alkyl, branched or unbranched C₁₋₄-perfluoroalkoxy, branched or unbranched C₁₋₄-perfluoroalkyl, oxo, amino, carboxy, amido, cyano, nitro, -SO₂NH₂, -CO-C₁₋₄-alkyl,-SO-C₁₋₄-alkyl, -SO₂-C₁₋₄-alkyl, -NH-SO₂-C₁₋₄-alkyl, wherein the C₁₋₄-alkyl may in each case be branched or unbranched, and a phenyl group, more preferably be selected from the group consisting of hydroxy, F, Cl, Br, methyl, ethyl, methoxy, ethoxy, oxo, CF₃ and a phenyl group.

If one or more of the residues R³-R⁸ represents or comprises a cycloaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteratoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of cyclopropyl, cyclobutyl, cyclopentyl, cyclohexyl, cycloheptyl, cyclooctyl, cyclopentenyl, cyclohexenyl, cycloheptenyl, cyclooctenyl, pyrrolidinyl, piperidinyl, piperazinyl, homo-piperazinyl and morpholinyl.

If one or more of the residues R³-R⁸ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched C₁₋₆-alkoxy, branched or unbranched C₁₋₆-alkyl, branched or unbranched C₁₋₄-perfluoroalkoxy, branched or unbranched C₁₋₄-perfluoroalkyl, amino, carboxy, oxo, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein the C₁₋₄-alkyl may in each case be branched or unbranched, and a phenyl group, more preferably be selected from the group consisting of hydroxy, F, Cl, Br, methyl, ethyl, methoxy, ethoxy, CF₃, oxo and a phenyl group.

If one or more of the residues R¹-R⁸ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched C₁₋₆-alkyl group, a linear or branched C₁₋₆ alcoxy group, a formyl group, a hydroxy group, a trifluoromethyl group, a trifluoromethoxy group, a -CO-C₁₋₆-alkyl group, a cyano group, a nitro group, a carboxy group, a -CO-O-C₁₋₆-alkyl group, a -CO-NR^{A}R^{B}- moiety, a -CO-NH-NR^{C}R^{D}-moiety, an ―SH, an -S-C₁₋₆-alkyl group, an -SO-C₁₋₆-alkyl group, an -SO₂-C₁₋₆-alkyl group, a -C₁₋₆-alkylene-S-C₁₋₆-alkyl group, a -C₁₋₆-alkylene-SO-C₁₋₆-alkyl group, a -C₁₋₆-alkylene-SO₂-C₁₋₆-alkyl group, an ―NH₂-moiety, an NHR'-moiety or an NR'R"-moiety, wherein R' and R" independently represent a linear or branched C₁₋₆-alkyl group, a C₁₋₆-alkyl group substituted by one or more hydroxy groups and a ―C₁₋₆-alkylene-NR^{E}R^{F} group,
whereby R^{A}, R^{B}, identical or different, represent hydrogen or a C₁₋₆-alkyl group, or R^{A} and R^{B} together with the bridging nitrogen atom form a saturated, mono- or bicyclic, 3-10 membered heterocyclic ring system, which may be at least mono-substituted by one or more, identical or different, C₁₋₆ alkyl groups and/or which may contain at least one further heteroatom selected from the group consisting of nitrogen, oxygen and sulphur as a ring member,

R^{C}, R^{D}, identical or different, represent a hydrogen atom, a C₁₋₆-alkyl group, a -CO-O-C₁₋₆-alkyl group, a C₃₋₈-cycloalkyl group, a C₁₋₆-alkylene-C₃₋₈-cycloalkyl group, C₁₋₆-alkylene-O-C₁₋₆-alkyl group or a C₁₋₆-alkyl group substituted with one or more hydroxy groups, or R^{C}, R^{D} together with the bridging nitrogen atom form a saturated, mono- or bicyclic, 3-10 membered heterocyclic ring system, which may be at least mono-substituted by one or more substituents independently selected from the group consisting of C₁₋₆ alkyl group, a -CO-C₁₋₆-alkyl group, a -CO-O- C₁₋₆-alkyl group, a - CO-NH- C₁₋₆-alkyl group, a -CS-NH- C₁₋₆-alkyl group, an oxo group, a C₁₋₆-alkyl group substituted with one or more hydroxy groups, a C₁₋₆-alkylene-O-C₁₋₆-alkyl group and a -CO-NH₂ group and/or which may contain at least one further heteroatom selected from the group consisting of nitrogen, oxygen and sulphur as a ring member, and

wherein R^{E}, R^{F}, identical or different, represent hydrogen or a C₁₋₆-alkyl group, or R^{E} and R^{F} together with the bridging nitrogen atom form a saturated, mono- or bicyclic, 3-10 membered heterocyclic ring system, which may be at least mono-substituted by one or more, identical or different C₁₋₆ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues R³-R⁸ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched C₁₋₆-alkyl group, a linear or branched C₁₋₆ alcoxy group, a formyl group, a hydroxy group, a trifluoromethyl group, a trifluoromethoxy group, a -CO-C₁₋₆-alkyl group, a cyano group, a carboxy group, a ―CO-O-C₁₋₆-alkyl group, a -CO-NR^{A}R^{B}- moiety, a -CO-NH-NR^{C}R^{D}-moiety, an -S-C₁₋₆-alkyl group, an -SO-C₁₋₆-alkyl group, an -SO₂-C₁₋₆-alkyl group, a -C₁₋₆-alkylene-S-C₁₋₆-alkyl group, a -C₁₋₆-alkylene-SO-C₁₋₆-alkyl group, a -C₁₋₆-alkylene-SO₂-C₁₋₆-alkyl group, a C₁₋₆-alkyl group substituted by one or more hydroxy groups and a ―C₁₋₆-alkylene-NR^{E}R^{F} group,

whereby R^{A}, R^{B}, identical or different, represent hydrogen or a C₁₋₆-alkyl group, or R^{A} and R^{B} together with the bridging nitrogen atom form a saturated, mono- or bicyclic, 3-10 membered heterocyclic ring system, which may be at least mono-substituted by one or more, identical or different, C₁₋₆ alkyl groups and/or which may contain at least one further heteroatom selected from the group consisting of nitrogen, oxygen and sulphur as a ring member,

R^{C}, R^{D}, identical or different, represent a hydrogen atom, a C₁₋₆-alkyl group, a -CO-O-C₁₋₆-alkyl group, a C₃₋₈-cycloalkyl group, a C₁₋₆-alkylene-C₃₋₈-cycloalkyl group, C₁₋₆-alkylene-O-C₁₋₆-alkyl group or a C₁₋₆-alkyl group substituted with one or more hydroxy groups, or R^{C}, R^{D} together with the bridging nitrogen atom form a saturated, mono- or bicyclic, 3-10 membered heterocyclic ring system, which may be at least mono-substituted by one or more substituents independently selected from the group consisting of C₁₋₆ alkyl group, a -CO-C₁₋₆-alkyl group, a -CO-O-C₁₋₆-alkyl group, a - CO-NH- C₁₋₆-alkyl group, a -CS-NH- C₁₋₆-alkyl group, an oxo group, a C₁₋₆-alkyl group substituted with one or more hydroxy groups, a C₁₋₆-alkylene-O-C₁₋₆-alkyl group and a -CO-NH₂ group and/or which may contain at least one further heteroatom selected from the group consisting of nitrogen, oxygen and sulphur as a ring member, and

wherein R^{E}, R^{F}, identical or different, represent hydrogen or a C₁₋₆-alkyl group, or R^{E} and R^{F} together with the bridging nitrogen atom form a saturated, mono- or bicyclic, 3-10 membered heterocyclic ring system, which may be at least mono-substituted by one or more, identical or different C₁₋₆ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members. Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo[2,1-b]thiazolyl, triazolyl and pyrazolyl.

If one or more of the residues R⁴-R⁸ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein the C₁₋₄-alkyl may in each case be branched or unbranched, and a phenyl group, more preferably be selected from the group consisting of hydroxy, F, Cl, Br, methoxy, ethoxy, CF₃ and a phenyl group.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues R⁴-R⁸ represents or comprises a linear or branched alkylene group, said alkylene group may preferably be selected from the group consisting of ― methylene -(CH₂)-, ethylene -(CH₂-CH₂)-, n-propylene -(CH₂-CH₂-CH₂)- or isopropylene ―(-C(CH₃)₂)-.

Preferred are substituted pyrazoline compounds produced of general formula I given above, wherein

R¹ represents an optionally at least mono-substituted phenyl group,

R² represents an optionally at least mono-substituted phenyl group,

R³ represents a saturated or unsaturated, optionally at least mono-substituted, optionally at least one heteroatom as ring member containing cycloaliphatic group, which may be condensed with an optionally at least mono-substituted mono- or polycyclic ring system, or R³ represents an optionally at least mono-substituted aryl or heteroaryl group, which may be condensed with an optionally at least mono-substituted mono- or polycyclic ring system, or R³ represents an ―NR⁴R⁵-moiety,

R⁴ and R⁵, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an ―SO₂-R⁶-moiety, or an -NR⁷R⁸-moiety,

R⁶ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R⁷ and R⁸, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,

whereby preferably the following provisos (disclaimers) apply:
that R⁴ and R⁵ do not both represent a hydrogen atom, and that if one of the residues R⁴ and R⁵ represents a hydrogen atom or a linear or branched, saturated or unsaturated, substituted or unsubstituted aliphatic group, the other one of these residues R⁴ and R⁵ does not represent a substituted or unsubstituted pyridyl group, a substituted or unsubstituted pyrimidyl group, a substituted or unsubstituted pyridazyl group, a substituted or unsubstituted pyrazinyl group, a substituted or unsubstituted thienyl group, a substituted or unsubstituted benzyl group, a substituted or unsubstituted phenethyl group, a substituted or unsubstituted phenyl group, a substituted or unsubstituted phenyl group, which is condensed (attached) to at least one, optionally substituted ring or ringsystem, an -NH-phenyl-moiety, wherein the phenyl group may be at least mono-substituted, an unsubstituted or substituted thiazole group, or an unsubstituted or substituted [1,3,4]thiadiazole group.

Preferred are also substituted pyrazoline compounds produced of general formula I given above, wherein R¹ represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R", whereby R' and R" for each substituent independently represent linear or branched C₁₋₆ alkyl, preferably R¹ represents a phenyl group, which is optionally substituted by one or more substituents selected from the group consisting of methyl, ethyl, F, Cl, Br and CF₃, more preferably R¹ represents a phenyl group, which is substituted with a chlorine atom in the 4-position, and R²-R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are substituted pyrazoline compounds produced of general formula I given above, wherein R² represents a phenyl group, which is optionally substituted by one or more substituents independently selected from the group consisting of a linear or branched C₁₋₆-alkyl group, a linear or branched C₁₋₆-alkoxy group, a halogen atom, CH₂F, CHF₂, CF₃, CN, OH, NO₂, -(C=O)-R', SH, SR', SOR', SO₂R', NH₂, NHR', NR'R", -(C=O)-NH₂, -(C=O)-NHR' and -(C=O)-NR'R" whereby R' and R" for each substituent independently represent linear or branched C₁₋₆ alkyl, preferably R² represents a phenyl group, which is optionally substituted by one or more substituents selected from the group consisting of methyl, ethyl, F, Cl, Br and CF₃, more preferably R² a phenyl group, which is di-substituted with two chlorine atoms in the 2- and 4-position, and R¹ and R³-R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Preference is also given to substituted pyrazoline compounds produced of general formula I given above, wherein R³ represents a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or R³ represents an optionally at least mono-substituted, 5- or 6-membered aryl or heteroaryl group, which may be condensed with an optionally at least mono-substituted mono- or polycyclic ring system, or R³ represents an -NR⁴R⁵-moiety, preferably R³ represents a saturated, optionally at least mono-substituted, optionally one or more nitrogen-atoms as ring member containing C₃₋₈ cycloaliphatic group, which may be condensed with an optionally at least mono-substituted mono- or polycyclic ring system, or R³ represents an -NR⁴R⁵-moiety, more preferably R³ represents a pyrrolidinyl group, a piperidinyl group or a piperazinyl group, whereby each of these groups may be substituted with one or more C₁₋₆-alkyl groups, or R³ represents an ―NR⁴R⁵-moiety and R¹, R² and R⁴-R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Furthermore, substituted pyrazoline compounds produced of general formula I given above are preferred, wherein R⁴ and R⁵, identical or different, represent a hydrogen atom, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-CH₂-) or ethylene (-CH₂-CH₂)-group, an -SO₂-R⁶-moiety, or an -NR⁷R⁸-moiety, preferably one of these residues R⁴ and R⁵ represents a hydrogen atom and the other one of these residues R⁴ and R⁵ represents a saturated or unsaturated, optionally at least mono-substituted, optionally at least one heteroatom as ring member containing C₃₋₈-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an -SO₂-R⁶-moiety, or an -NR⁷R⁸-moiety, or R⁴ and R⁵, identical or different, each represent a C₁₋₆ alkyl group, more preferably one of these residues R⁴ and R⁵ represents a hydrogen atom and the other one of these residues R⁴ and R⁵ represents an optionally at least mono-substituted pyrrolidinyl group, an optionally at least mono-substituted piperidinyl group, an optionally at least mono-substituted piperazinyl group, an optionally at least mono-substituted triazolyl group, an -SO₂-R⁶-moiety, or an -NR⁷R⁸-moiety, or R⁴ and R⁵, identical or different, represent a methyl group, an ethyl group, an n-propyl group, an isopropyl group, an n-butyl group, a sec-butyl group or a tert.-butyl group, and R¹-R³ and R⁶-R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are substituted pyrazoline compounds produced of general formula I given above, wherein R⁶ represents a linear or branched, saturated or unsaturated, optionally at least mono-substituted C₁₋₆ aliphatic group, a saturated or unsaturated, optionally at least mono-substituted, optionally at least one heteroatom as ring member containing C₃₋₈ cycloaliphatic group, which may be condensed with a mono-or polycyclic ring-system, or an optionally at least mono-substituted, 5- or 6-membered aryl or heteroaryl group, which may be condensed with a mono- or polycyclic ring system and/or bonded via a methylene (-CH₂-) or ethylene (-CH₂-CH₂)-group, preferably R⁶ represents a C₁₋₆-alkyl group, a saturated, optionally at least mono-substituted cycloaliphatic group, which may be condensed with a mono-or polycyclic ring-system, or a phenyl group, which is optionally substituted with one or more C₁₋₆ alkyl groups, and R¹-R⁵, R⁷ and R⁸ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Moreover substituted pyrazoline compounds produced of general formula I given above are preferred, wherein R⁷ and R⁸, identical or different, represent a hydrogen atom, an unbranched or branched, saturated or unsaturated, optionally at least mono-substituted C₁₋₆ aliphatic radical, a saturated or unsaturated, optionally at least mono-substituted, optionally at least one heteroatom as ring member containing C₃₋₈ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene (-CH₂-) or ethylene (-CH₂-CH₂)-group, preferably represent a hydrogen atom or a C₁₋₆ alkyl radical, and R¹-R⁶ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Particularly preferred are compounds produced of general formula I given below, 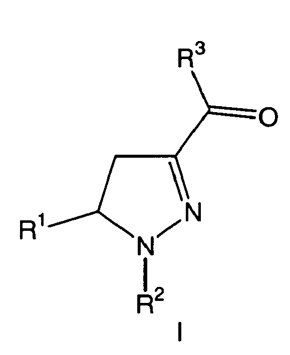 wherein

R¹ represents a phenyl ring, which is mono-substituted with a halogen atom, preferably a chlorine atom, in its 4-position,

R² represents a phenyl ring, which is di-substituted with two halogen atoms, preferably chlorine atoms, in its 2- and 4-position,

R³ represents a pyrrolidinyl group, a piperidinyl group, a piperazinyl group, a homo-piperazinyl group, a morpholinyl group, or an ―NR⁴R⁵-moiety,

R⁴ represents a hydrogen atom or a linear or branched C₁₋₆-alkyl group,

R⁵ represents a linear or branched C₁₋₆ alkyl group, a pyrrolidinyl group, a piperidinyl group, a piperazinyl group, a homo-piperazinyl group, a morpholinyl group, a triazolyl group, whereby each of the heterocyclic rings may be substituted with one or more, identical or different, C₁₋₆-alkyl groups, or an -SO₂-R⁶-moiety, and

R⁶ represents a phenyl group, which is optionally substituted with one or more C₁₋₆ alkyl groups, which may be identical or different,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Most particularly preferred are substituted pyrazoline compounds produced selected from the group consisting of:
N-piperidin-1-yl-5-(4-chloro-phenyl)-1-(2,4-dichlorophenyl)-4,5-dihydro-1H-pyrazole-3-carboxamide,
5-(4-chloro-phenyl)-1-(2,4-dichloro-phenyl)-4,5-dihydro-1H-pyrazole-3-carboxylic acid-[1,2,4]-triazole-4-yl-amide,
5-(4-chloro-phenyl)-1-(2,4-dichloro-phenyl)-4,5-dihydro-1H-pyrazole-3-carboxylic acid-(4-methyl-piperazin-1-yl)-amide,
5-(4-chloro-phenyl)-1-(2,4-dichloro-phenyl)-4,5-dihydro-1H-pyrazole-3-carboxylic acid diethylamide,
[5-(4-chloro-phenyl)-1-(2,4-dichloro-phenyl)-4,5-dihydro-1H-pyrazole-3-yl]-piperidine-1-yl-methanone,
N-[5-(4-chloro-phenyl)-1-(2,4-dichlorophenyl)-4,5-dihydro-1H-pyrazole-3-carbonyl]-4-methylphenylsufonamide,
   optionally in the form of a corresponding N-oxide, or a corresponding salt, or a corresponding solvate.

In another aspect the present invention provides novel compounds for use as active substances in medicaments, which are suitable for the regulation especially the reduction of triglyceride levels in the blood plasma.

Said object was achieved by providing the substituted pyrazoline compounds of general formula II given below, their stereoisomers, corresponding salts and corresponding solvates thereof.

It has been found that these compounds have a marked effect on the level of triglycerides in the blood plasma.

Thus, in another aspect the present invention relates to substituted pyrazoline compounds produced of general formula (III) 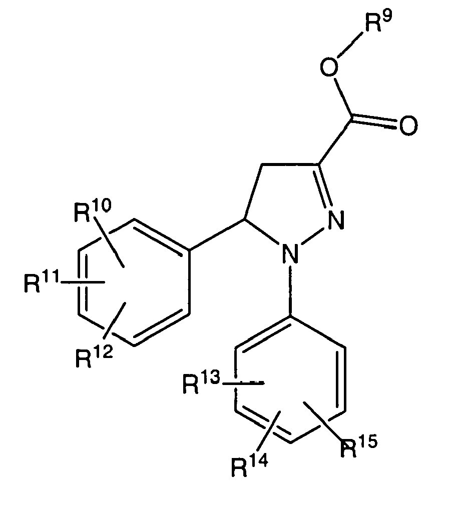 wherein

R⁹ represents hydrogen or a linear or branched C₁₋₄-alkyl group,

R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,

R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;

R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

It is very preferred if to these compounds produced according to formula III the following proviso (disclaimer) applies:
with the proviso (disclaimer) that
if R⁹ and R¹³ are H and R¹⁴ and R¹⁵ both represent Cl in the 3- and 4-position
of the phenyl ring neither of R¹², R¹³ and R¹⁴ may represent F in the 4-position
of the phenyl ring if the other two of R¹², R¹³ and R¹⁴ both represent H.

These compounds produced had a surprising effect on the blood levels of diet relevant substances, e.g. Triglycerides.

Preferred linear or branched, saturated or unsaturated aliphatic groups, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl, n-decyl, vinyl, ethinyl, propenyl, propinyl, butenyl and butinyl.

In the context of this invention, alkyl and cycloalkyl radicals are understood as meaning saturated and unsaturated (but not aromatic), branched, unbranched and cyclic hydrocarbons, which can be unsubstituted or mono- or polysubstituted. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cydoalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also CHF₂, CF₃ or CH₂OH) as well as pyrazolinone, oxopyrazolinone, [1,4]-dioxane or dioxolane.

Here, in connection with alkyl and cycloalkyl - unless expressly defined otherwise - the term substituted in the context of this invention is understood as meaning replacement of at least one hydrogen radical by F, Cl, Br, I, NH₂, SH or OH, "polysubstituted" radicals being understood as meaning that the replacement takes effect both on different and on the same atoms several times with the same or different substituents, for example three times on the same C atom, as in the case of CF₃, or at different places, as in the case of -CH(OH)-CH=CH-CHCl₂. Particularly preferred substituents here are F, Cl and OH. In respect of cycloalkyl, the hydrogen radical can also be replaced by OC₁₋₃-alkyl or C₁₋₃-alkyl (in each case mono- or polysubstituted or unsubstituted), in particular methyl, ethyl, n-propyl, i-propyl, CF₃, methoxy or ethoxy.

The term (CH₂)₃₋₆ is to be understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a CF₃, a CN, an NO₂, an NRR, a C₁₋₆-alkyl (saturated), a C₁₋₆-alkoxy, a C₃₋₈-cycloalkoxy, a C₃₋₈-cydoalkyl or a C₂₋₆-alkylene, with R being H or C₁₋₆-alkyl.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions. Especially this covers any "physiologically acceptable salt".

The term "physiologically acceptable salt" to be understood as being equivalent and interchangeable with "physiologically acceptable salt" means in the context of this invention any salt that is physiologically tolerated (most of the time meaning not being toxic- especially not caused by the counter-ion) if used appropriately for a treatment especially if used on or applied to humans and/or mammals.
These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of:
hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound produced according to the invention in which this compound has attached to it via non-covalent binding another molecule (most likely a polar solvent) especially including hydrates (mono- and multihydrates) and alcoholates, e.g. methanolate and ethanolate.

The term "salt" is to be understood as meaning any form of the active compound produced used according to the invention in which it assumes an ionic form or is charged and is coupled with a counter-ion (a cation or anion) or is in solution. By this are also to be understood complexes of the active compound produced with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds produced used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds produced used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

Unless otherwise stated, the compounds produced of the invention are also meant to include compounds produced which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

### General procedures

This invention relates to methods for the preparation of 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxamide (II), as well as the intermediates to this compound. 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxamide can be obtained by the reaction of an activated carbonyl compound derived from 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxylic acid (IIIa) with *N*-aminopiperidine in the presence or absence of an acid acceptor or suitable condensing agent.

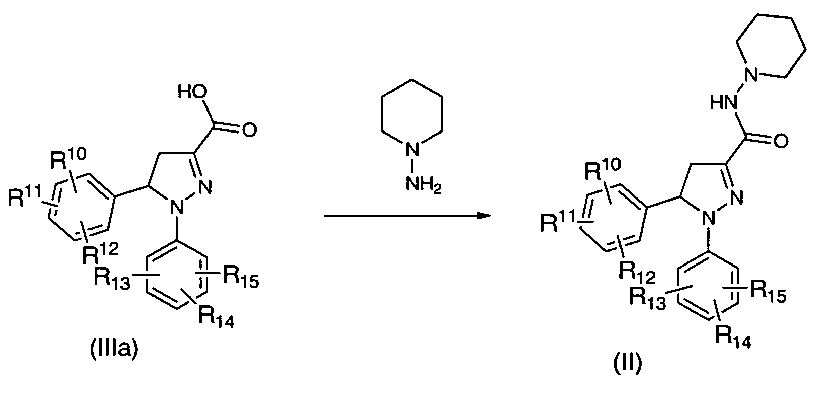

The carboxylic group may be activated by the introduction of a suitable leaving group according to conventional methods well known to those skilled in the art. Preferably the carboxylic acid can be transformed into the acid chloride, an acid anhydride, a mixed anhydride, a straight or branched C₁₋₄ alkyl ester or an activated ester such as p-nitrophenylester. Other well known methods for the activation of acids include the activation with Lewis acids, inorganic or organic acids, such as H₂SO₄ or TsOH, carbodiimides such as N,N-dicyclohexylcarbodiimide or 1-ethyl-3-(3-dimethylaminopropyl)-carbodiimide (EDC), carbonyldiimadazole, O-Benzotriazole-N,N,N',N'-tetramethyl-uronium-hexafluoro-phosphate (HBTU) or benzotriazol-N-oxotris(dimethylamino) phosphonium hexafluorophosphate (BOP)), among others.

One particularly useful method involves the chlorination or bromination of 5-(4-chlorophenyl)-1-(2,4-dichlorophenyl)-4,5-dihydro-1H-pyrazole-3-carboxylic acid (IIIa: R¹⁰=R¹¹=R¹³=H, R¹²=R¹⁴=4-chloro and R¹⁵=2-chloro) with thionyl chloride or another chlorinated or brominated agent, followed by treatment with *N*-aminopiperidine in the presence of an acid acceptor such as an amine base, preferably *N,N-*diisopropylethylamine or triethylamine. Suitable solvents for the chlorination reaction are those which are inert to hydrogen chloride, preferably toluene. Preferred temperatures for this process are between 70 and 90 ºC. A catalytic amount of *N,N-*dimethylformamide can be added to the rection mixture to improve the rate and the yield.

The condensation of compound with general formula (IIIa) with *N*-aminopiperidine can also be carried out in an inert atmosphere with 1-ethyl-3-(3-dimethylaminopropyl)carbodiimide (EDC) and 1-hydroxybenzotriazole (HOBt) in the presence or absence of N-methylmorpholine, in an aprotic solvent, such as N,N-dimethylformamide or tetrahydrofuran, and in the presence of a base, such as triethylamine (Tetrahedron Lett. 45 (2004) 4977-4980). The reaction is preferably carried out at room temperature for 15-24 hours. The use of a polymer-supported EDC (P-EDC) is also a suitable method for this condensation [Tetrahedron Letters 39 (1998) 1487-1488, Tetrahedron Letters 43(48), 7685-7688 (1993)].

The coupling of a compound of general formula (IIIa) with *N*-aminopiperidine can be achieved using HBTU as a condensating agent in the presence of a base, such as DIPEA and an aprotic solvent, such as acetonitrile, preferably at room temperature and during 15-24 hours.

Alternately, the mixed anhydride of a compound of general formula (IIIa) can be prepared by reaction of ethylchloroformate with the acid (IIIa) in the presence of a base such as triethylamine and then reacted with 1-aminopiperidine in a solvent such as dichloromethane in an inert atmosphere, at a temperature between 0 ºC and room temperature, in the presence of a base such as triethylamine or *N*,*N-*diisopropylethylamine.

The compound of general formula (IIIa) can be obtained by a variety of methods, which are described below. In all these schemes, although normally the reaction is shown for the ester intermediates, the reactions are also possible with the acid itself or with the compound with an acid equivalent group, for example, a cyano group, which can be converted into the acid by conventional methods.

1) The first method goes through the 4-(R¹⁰,R¹¹,R¹²-phenyl)-2-oxobut-3-enoic acid intermediate (IVa) or a ester derived from it (IVb) or a compound which contains an acid equivalent group instead of the carboxylic group (IVc), which reacts with phenylhydrazine (R¹³,R¹⁴R¹⁵ phenyl substituted) to give the desired 5-(R¹⁰,R¹¹,R¹²-phenyl)-1-(R¹³R¹⁴R¹⁵-phenyl)-N-(piperidin-1-yl)-4,5-dihydro-1H-pyrazole-3-carboxylic acid (IIIa). 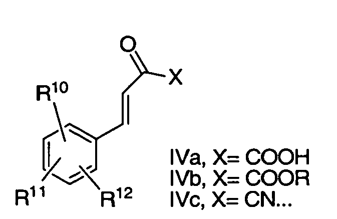 (E)-4-(R¹⁰,R¹¹,R¹²-phenyl)-2-oxobut-3-enoic acid intermediate (IV) can be isolated or generated in situ to be transformed into carboxylic acid (IIIa) without isolation, as shown in the following schemes. a) 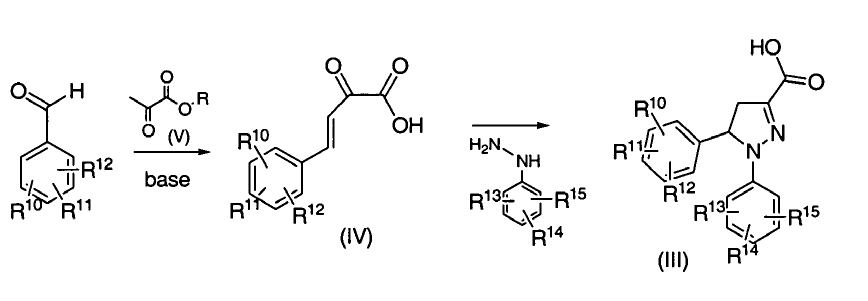 In this case, intermediate (IV) is formed and isolated by the condensation of (R¹⁰,R¹¹,R¹²)-benzaldehyde with the 2-oxo-propanoic acid (V) or an ester or salt derivative, and is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used. Preferably the reaction is carried out in a protic reaction medium such as a C₁₋₄ alkyl alcohol or mixtures of these. Mixtures of such alcohols with water, e.g. ethanol/water may also be used. Reaction temperature as well as the reaction time may vary over a broad range. Preferred reaction temperatures range from -10 °C to the boiling point of the reaction medium. Suitable reaction times may be between 12-15 hours. Most preferred temperatures are between 0-25 ºC. Also preferably, the reaction of the (R¹⁰,R¹¹,R¹²)-benzaldehyde with sodium pyruvate is carried out under acid catalysed conditions, more preferably by refluxing the mixture in dichloromethane in the presence of copper(II)trifluoromethanesulfonate as described, for example, in Synlett, (1), 147-149, 2001.

The reaction of intermediate (IV) with phenylhydrazine (R¹³,R¹⁴,R¹⁵ phenyl substituted) is preferably carried out in a suitable reaction medium such as C₁₋₄-alcohols or ethers such as dioxane or tetrahydrofuran or mixtures of at least two of these above mentioned compounds. Also preferably, said reaction may be carried out in the presence of an acid, whereby the acid may be organic such as acetic acid and/or inorganic such as hydrochloric acid. Reaction temperature as well as the reaction time may vary over a broad range. Suitable reaction temperatures range from room temperature, i.e. approximately 25 °C to the boiling point of the reaction medium. Suitable reaction times may vary for example from several minutes to several hours, preferably 1-2 h.

b)

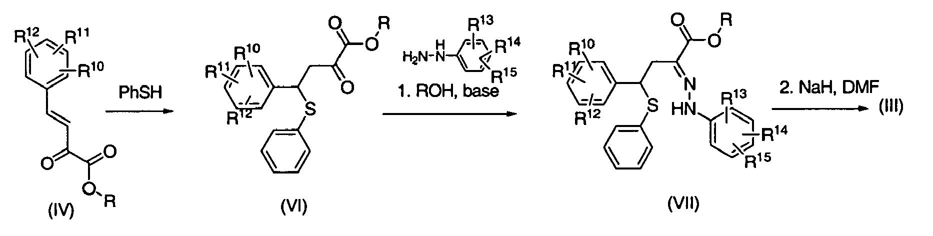

In this case, (E)-4-(R¹⁰,R¹¹,R¹²-phenyl)-2-oxobut-3-enoic acid or a derivative (IV) is further derivatized by a Michael addition to a phenylthio analog (VI), which can react with phenylhydrazine (R¹³,R¹⁴,R¹⁵ phenyl subtituted) to form the acid (Illa) in two steps. Tetrahedron 81 (2005) 5235-5240; Tetrahedron Asymmetry 12 (2001) 1923-1928. The Michael addition can be performed in an aprotic and dry solvent, such as toluene, in the presence of a catalytic amount of an organic base and tiophenol. The reaction is preferred carried out at -20 ºC for 4-24 hours. The Michael adduct (VI) is placed in a protic solvent, such as methanol, with a catalytic amount of a base such as KHSO₄ and phenylhydrazine (R¹³,R¹⁴,R¹⁵ phenyl subtituted) to form the hydrazone (VII). The preferred temperature for this reaction is between 25-50 ºC and it is kept for 4-15 hours. The intramolecular cyclization of the hydrazone is carried out in an aprotic and dry solvent, such as DMF, under an inert atmosphere in the presence of a base, such as NaH. If the starting material is an ester or has an acid equivalent group, such as cyano, instead of the carboxylic group, a saponification or hydrolytic step is needed at the end.

c) 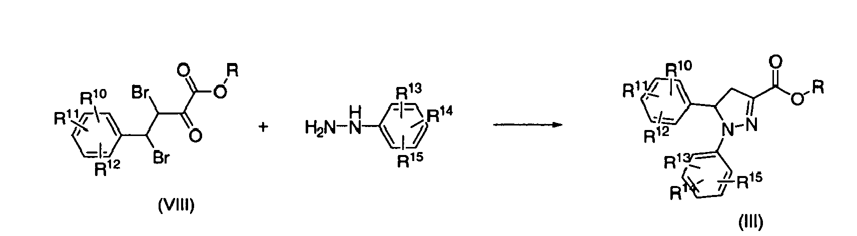 The cyclization with phenylhydrazine (R¹³,R¹⁴,R¹⁵ phenyl subtituted) can also be performed starting from the dibromide derivative of (E)-4-(R¹⁰,R¹¹,R¹²-phenyl)-2-oxobut-3-enoic acid, 3,4-dibromo-4-(R¹⁰,R¹¹,R¹²-phenyl)-2-oxobutanoic acid (VIII), obtained by a bromination of the acid (IV) using methods described in the literature. Tetrahedron Lett. 1998, 39(44), 8163-8166; J. Chem. Soc., Perkin Trans 1, 1999, 21, 3069-3070; Tetrahedron, 1999, 55(36), 11127-11142; J. Heterocyclic Chem. 1986, 23, 1199. Typical conditions for the bromination are those which use an aprotic solvent, such as dichloromethane, and bromine at room temperature for 1-2 hours. Preferred conditions for the cyclization are in refuxing DMF for several hours, most preferably for 2-3 hours or in a mixture of dioxane, water and acetic acid, among others. Chemistry of Heterocyclic Compounds, 33(6), 1997; Indian J. Chem. 20B, 1090 (1981); Indian J. Chem. 29B, 887 (1990).

d) 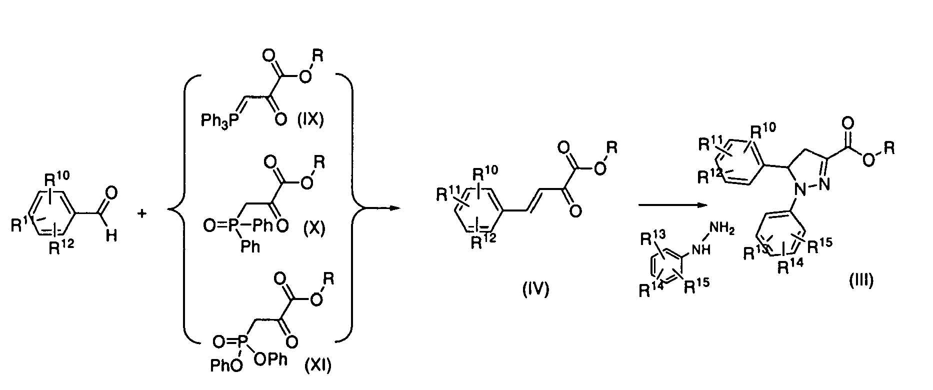 Another possibility that goes through the intermediate (IV) is the reaction between (R¹³,R¹⁴,R¹⁵)-benzaldehyde and a phosphonium ylide (IX), a phosphine oxide (X) or a phosphonate (XI) by a Wittig-type reaction, followed by the condensation and cyclization with phenylhydrazine (R¹³,R¹⁴,R¹⁵ phenyl subtituted). Tetrahedron 1994, 50(44), 12727-12742; Zhurnal Obshchei Khimii, 56(2), 347-353, 1986. The Wittig-type reaction can be performed in an aprotic solvent, such as tetrahydrofuran, using a base, such as NaH or LDA. The cyclization in the presence of phenylhydrazine (R¹³,R¹⁴,R¹⁵ phenyl substituted) can be carried out using the same conditions described in section a) above. Another way to intermediate (IV) that uses phosphonates is shown below. J. Chem. Soc, Perkin Trans I, 1995(7), 741-742. 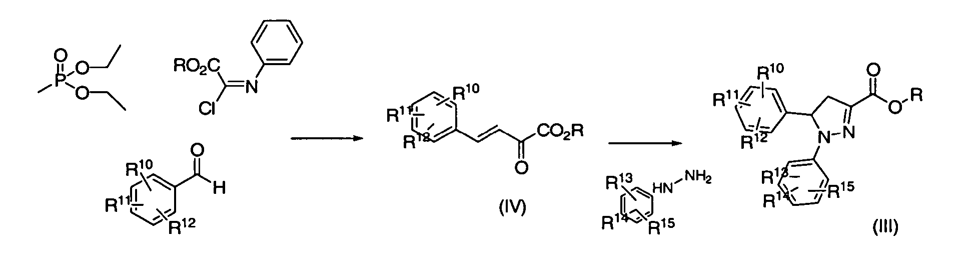 A typical procedure for this reaction consists of the addition of the phosphonate over a base such as butyllithium in a dry solvent, such as tetrahydrofuran at low temperature (preferably -70º C) and then *N*-phenylethoxycarbonylacetimidoyl chloride and the (R¹⁰,R¹¹,R¹²)-benzaldehyde are added, and the reaction is performed preferably at room temperature. The cyclization is performed as described above.

e) 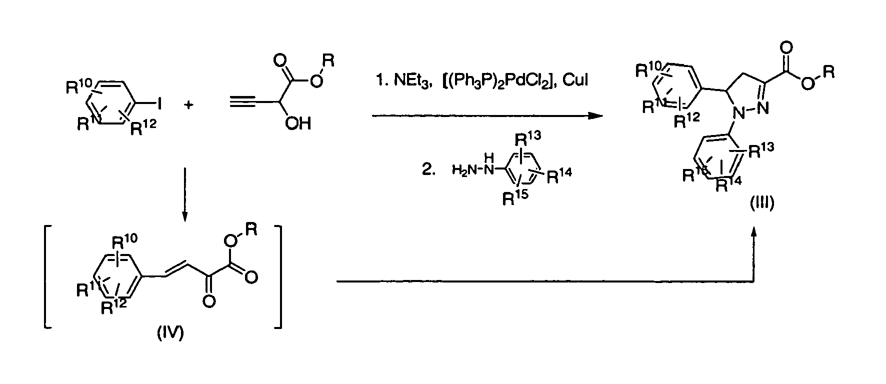 Intermediate (IV) can be also generated in-situ by an unusual Sonogashira coupling of iodobenzene (R¹⁰,R¹¹,R¹² phenyl subtituted) with 2-hydroxybut-3-ynoic acid, catalyzed by Pd and Cu and in the presence of a base, such as triethylamine, and in an aprotic and dry solvent, such as tetrahydrofuran. The preferred temperature for this reaction is the boiling point of the solvent. Phenylhydrazine (R¹³,R¹⁴,R¹⁵ phenyl subtituted) is added in a second step after 10-15 hours, but without isolating the intermediate (IV), and the mixture was kept heating at reflux for 5-10 hours. Angew. Chem. Int. Ed. 2000, 39(7), 1253-1256.

2) An alternate method for the preparation of 5-(R¹¹,R¹²,R¹³-phenyl)-1-(R¹³,R¹⁴,R¹⁵-phenyl)-N-(piperidin-1-yl)-4,5-dihydro-1H-pyrazole-3-carboxylic acid (IIIa) is the intramolecular Michael-type addition of the hydrazone (XII), formed from 2-(2-((R¹³,R¹⁴,R¹⁵)-phenyl)hydrazono)propanoic acid (XIII), by an α-alkylation of this hydrazone with (R¹⁰,R¹¹,R¹²)-benzaldehyde. J. Het. Chem. 12(4), 779-781, 1975; J. Chem. Enginneering Data, 29(2), 225-229, 1984; Indian J. Chem. 27B, 3, 245-249, 1988. The formation of hydrazone (XII) can be performed in boiling ethanol in the presence of sodium acetate or at room temperature in the presence of glacial acetic acid. The cyclization to the acid or a derivative (III) can be achieve in boiling ethanol or in boiling water. 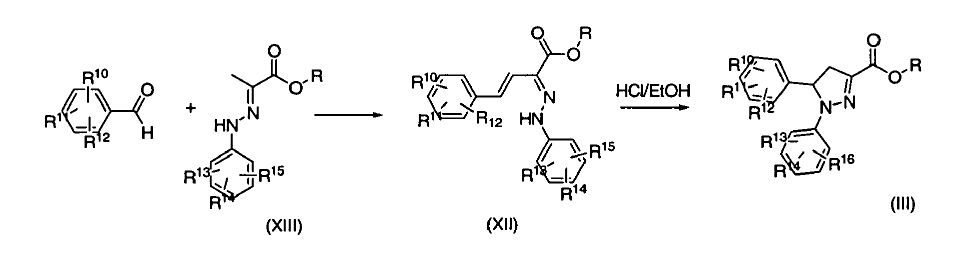 3) Another way to 5-(R¹⁰,R¹¹,R¹²-phenyl)-1-(R¹³,R¹⁴,R¹⁵)-phenyl)-N-(piperidin-1-yl)-4,5-dihydro-1H-pyrazole-3-carboxylic acid (IIIa) consists in the cycloaddition of the nitrilimine derived from 2-chloro or 2-bromo-2-(2-(R¹³,R¹⁴,R¹⁵)-phenyl)hydrazono)acetic acid (XIV) or a derivative (an ester or a compound with an acid equivalent group) with vinylbenzene (R¹⁰,R¹¹,R¹² phenyl substituted). Bull. Chem. Soc. Japan, 57(3), 787-790, 1984; Chem. Lett. 1982, 543-546. The reaction can be performed in an aprotic solvent, such as toluene or chloroform or in protic solvent, such as ethanol, and a base, such as triethylamine. Preferred temperatures are between room temperature and 120 º C 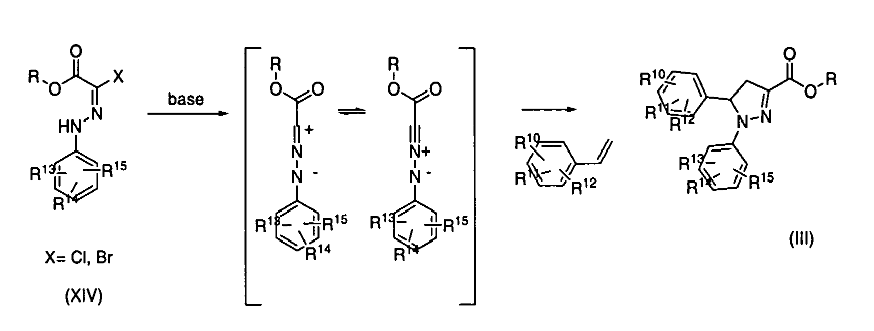 Alcoxycarbonyl-N-(R¹³,R¹⁴,R¹⁵-phenyl)hydrazonoyl chloride or bromide (XIV) can be prepared by halogenation of an ester of the hydrazone 2-(2-(R¹³,R¹⁴,R¹⁵-phenyl)hydrazono)acetic acid with a halogenating agent, such as NCS or NBS, in an appropriate solvent, such as DMF at room temperature. This hydrazone is readily accessible from an ester of 2-oxoacetic acid and phenylhydrazine (R¹³,R¹⁴,R¹⁵ phenyl subtituted) in refluxing ethanol and in the presence of some acetic acid and water. Synth. Commun. 2001, 31(1), 111-115; Tetrahedron, 1994, 50(25), 7543-7556. 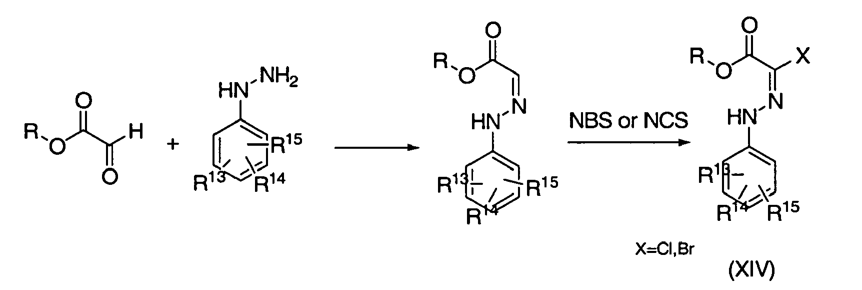 Alcoxycarbonyl-N-(R¹³,R¹⁴,R¹⁵-phenyl)hydrazonoyl chloride or bromide (XIV) can also be prepared by bromination of an ester of 2-(2-(R¹³,R¹⁴,R¹⁵-phenyl)hydrazono)-3-oxobutanoic acid in the presence of acetic acid and bromine, preferably at room temperature, which can be easily obtained from an ester of 3-oxobutanoic acid and the diazonium salt of (R¹³,R¹⁴,R¹⁵)-aniline. Synthesis, 1975, 333; J. Chem. Soc., Perkin Trans, 1, 1977, 2092. The diazonium salt of (R¹³,R¹⁴,R¹⁵⁾-aniline can be obtained by the addition of a solution of sodium nitrite in water over the (R¹³,R¹⁴,R¹⁵⁾-aniline in hydrochloric acid. The diazotization reaction in the presence of the ester of of 3-oxobutanoic acid can be carried out in the presence of sodium acetate, preferably at pH=4, at room temperature. 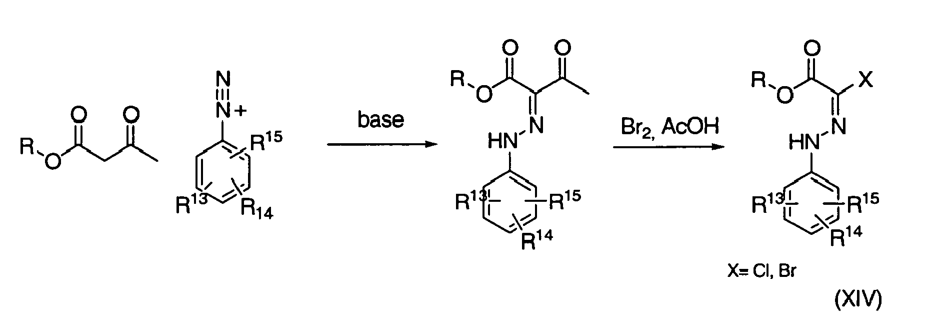 Another way to halohydrazone (XIV) is starting from an ester of 2-bromoacetic acid, the diazomium salt of (R¹³,R¹⁴,R¹⁵)-aniline and dimethylsulfide. Heterocycles, 1991, 32(6), 1101-1107. 2-bromoacetic acid and dimethylsulfide are refluxed in ethanol for about 30 min. The dimethylsulfonium bromide derivative can be isolated and then let to react with the diazonium salt of (R¹³,R¹⁴,R¹⁵⁾-aniline (obtained by the addition of a solution of sodium nitrite in water over this aniline in hydrochloric acid) in the presence of sodium acetate and acetic acid. Preferred temperatures are between 0 ºC and room temperature.

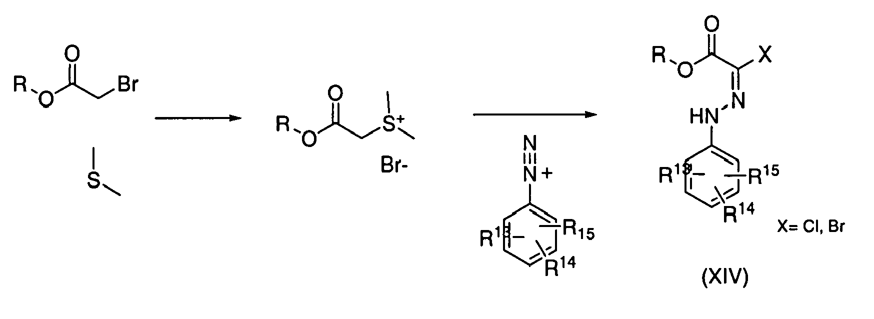

4) There are still other possibilities to obtain 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxylic acid (IIIa), by a thermolysis of the 2,3-dihydro-2,2,2-triphenyloxi-3-(R¹³,R¹⁴,R¹⁵-phenyl)-5-alcoxycarbonyl-1,3,4,2-oxadiazaphospholine (XV) in the presence of estyrene (R¹⁰,R¹¹,R¹² substituted). Chem. Lett. 1983, 507-510; Bull. Chem. Soc. Japan, 57(9), 2689-90, 1984. 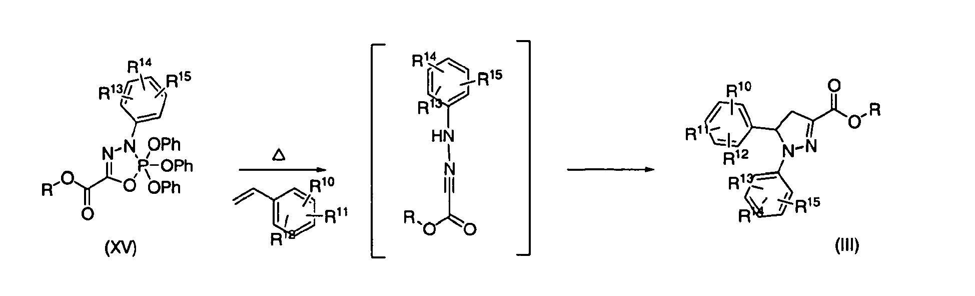 The reaction is preferably carried out in the presence of refluxing xylene for 12 or 24 hours. The starting phosphole can be prepared by the reaction of an ester of the hydrazide 2-(2-(R¹³,R¹⁴,R¹⁵-phenyl)hydrazinyl)-2-oxoacetic acid (XVI) with phosphorous pentachloride in toluene at room temperature, followed by the addition of a phenol, such as *O*-trimethylsilyl-p-cresol in refluxing toluene. 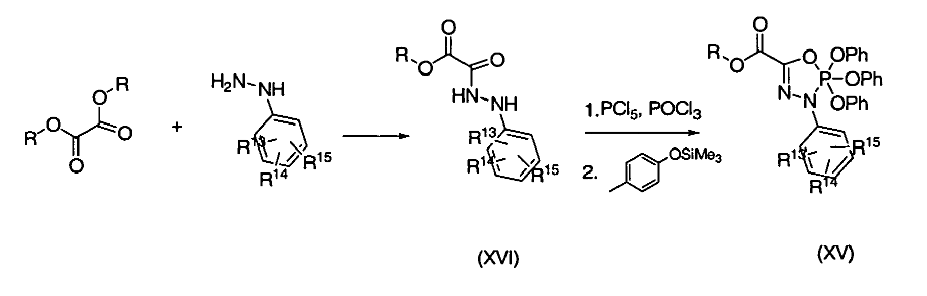 The hydrazide (XVI) can be easily obtained by the condensation of diethyl oxalate and phenylhydrazine (R¹³,R¹⁴,R¹⁵ phenyl substituted).5) Another suitable method for the obtention of 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxylic acid (IIIa) is consisting of the epoxidation of the intermediate (IV), 4-(R¹⁰,R¹¹,R¹²-phenyl)-2-oxobut-3-enoic acid, followed by a sequential nucleophilic attack of phenylhydrazine (R¹³,R¹⁴,R¹⁵ phenyl substituted) to the carbonyl and to the epoxide of this intermediate. The epoxidation can be performed with a variety of oxidant agents, such a perbenzoic acids, sodium peroxocarbonate, hydrogen peroxide, dioxiranes, hydroperoxides... The condensation with the hydrazine can be performed in refluxing ethanol to give 5-(R¹⁰,R¹¹,R¹²-phenyl)-1-(R¹³,R¹⁴,R¹⁵-phenyl)-4-hydroxy-4,5-dihydro-1 H-pyrazole-3-carboxylic acid (XVII). Then a radical deoxygenation of the secondary alcohol can be carried out by, for example, a Barton-McCombie reaction using xantates or thiocarbonates and hydrides. Synlett, 1990, 11, 705-706

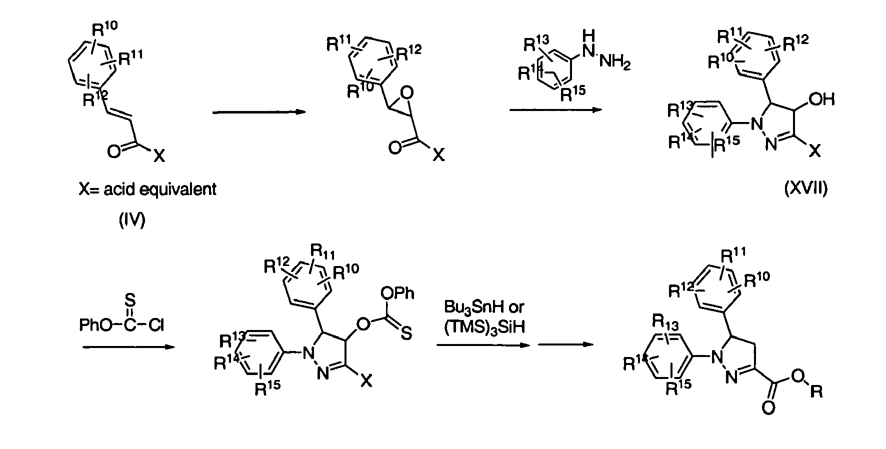

6) The acid 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxylic acid (Illa), can also be obtained by a Mannich reaction starting from phenylhydrazine (R¹³,R¹⁴,R¹⁵ phenyl substituted), (R¹⁰,R¹¹,R¹²)-benzaldehyde and a derivative of 2-oxopropanoic acid to give a derivative of 4-(R¹⁰,R¹¹,R¹²-phenyl)-4-(R¹³,R¹⁴,R¹⁵-phenylamino)-2-oxobutanoic acid (XVIII), followed by an electrophilic amination and a cyclization.

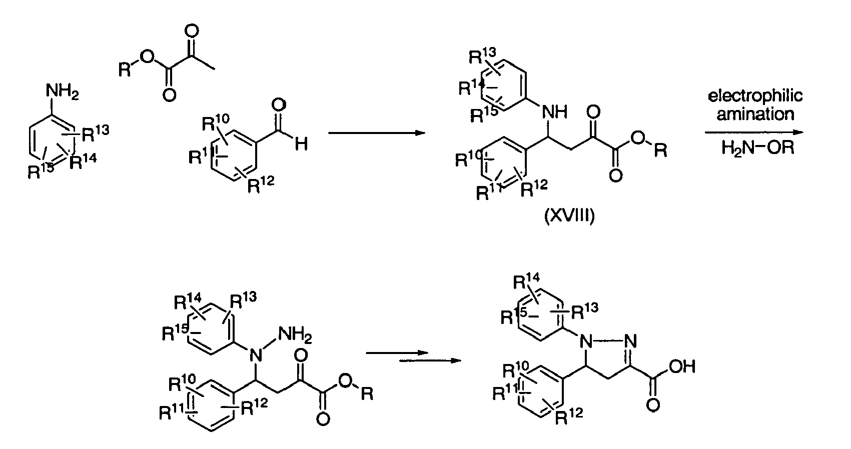

The electrophilic amination can be performed following the methodology of Friestad with a variety of *O*-substituted hydroxylamines J. Org. Chem. 2002, 67, 6237-6239 or by nitrosation using a variety of nitrites, followed by a reduction. The attack of the primary amine to the carbonyl gives the precursor of the desired acid (III).

In all the general procedures described above R¹⁰ to R¹⁵ has the meaning above mentioned and R is a branched or unbranched C₁₋₆ radical.

In another aspect the present invention relates to the process for obtaining 5-(4-chloro-phenyl)-1-(2,4-dichlorophenyl)-4,5-dihydro-1H-pyrazol-3-carboxylic acid 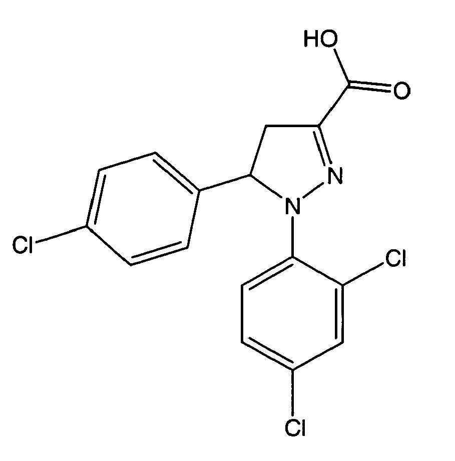 optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, in particular as an intermediate in a process for preparing substituted pyrazoline compounds of general formula (I).

In a further aspect the present invention also provides a process for the preparation of salts of substituted pyrazoline compounds of general formula (I) and stereoisomers thereof, wherein at least one compound of general formula (I) having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid. In yet a further aspect the present invention also provides a process for the preparation of salts of substituted pyrazoline compounds of general formula (I) or stereoisomers thereof, wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical. Suitable reaction media are, for example, any of the ones given above.

Solvates, preferably hydrates, of the substituted pyrazoline compounds of general formula (I), of corresponding stereoisomers, of corresponding N-oxides or of corresponding salts thereof may also be obtained by standard procedures known to those skilled in the art.

Substituted pyrazoline compounds produced of general formula I, which comprise nitrogen-atom containing saturated, unsaturated or aromatic rings may also be obtained in the form of their N-oxides by methods well known to those skilled in the art.

Those skilled in the art understand that the term substituted pyrazoline compounds as produced herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure. The purification and isolation of the inventive substituted pyrazoline compounds produced of general formula (I), of a corresponding stereoisomer, or salt, or N-oxide, or solvate or any intermediate thereof may, if required, be carried out by conventional methods known to those skilled in the art, e.g. chromatographic methods or recrystallization.

The substituted pyrazoline compounds produced of general formula (I) given below, their stereoisomers, corresponding N-oxides, corresponding salts thereof and corresponding solvates are toxicologically acceptable and are therefore suitable as pharmaceutical active substances for the preparation of medicaments.

In summary, the inventively produced pyrazoline compounds are distinguished by a broad spectrum of beneficial effects, while at the same time showing relatively little undesired effects, i.e. effects which do not positively contribute to or even interfere with the well being of the patient.

The present invention is illustrated below with the aid of examples. These illustrations are given solely by way of example and do not limit the general spirit of the present invention.

### Examples

### a) Preparation of 4-(4-chlorophenyl)-2-oxobut-3-enoic acid (IVa).

To a sodium hydroxide solution (7.11 g, 0.178 mol), in water (350 ml) and ethanol (15 ml), sodium pyruvate (43.1 g, 0.391 mol) is added in portions at 0-5 ºC. The mixture is stirred for 5 min. Then, a solution of 4-chlorobenzaldehyde (50 g, 0.356 mol) in ethanol (75 ml) is added, drop by drop, keeping the temperature between 0-5 ºC. The resulting suspension is stirred at this temperature for 6 hours. After that time, it is warmed to room temperature and is kept at 20-25 ºC for 6 additional hours. The evolution of the reaction is monitored by HPLC. Then, water (200 ml) was added to the crude and the mixture is cooled to 0-5 ºC. After that, concentrated hydrogen chloride (60 ml) was added to the mixture keeping the previous temperature. Once checked that pH is between 1-2, the mixture is stirred for at least 30 min, and then it is filtered off. The solid obtained by filtration is washed with water at room temperature (250 ml) and dried at 50-55 ºC. Then, the solid is resuspended in dichloromethane (304 ml), the solution is stirred for 1 h at room temperature and for an additional hour at 0-5 ºC. The solid is filtered, washed with cold dichloromethane (76 ml) and dried in a vacuum heat cabinet at 50 ºC to give a yellow solid (66.8 g, 89 % yield).

IR (KBr, cm⁻¹) : 3500-2500, 1719,3, 1686,5, 1603,4, 1587,8, 1081,9.

¹H RMN (CDCl₃, δ ppm) : 7,4 (d, J=8,4Hz, 2H), 7,5 (d, J=16,1 Hz, 1 H), 7,6 (d, J=8,4Hz, 2H), 8,1 (d, J=16,1 Hz, 1 H).

### b) Preparation of 5-(4-chlorophenyl)-1-(2,4-dichlorophenyl)-4,5-dihydro-1H-pyrazole-3-carboxylic acid (IIIa)

A suspension of 4-(4-chlorophenyl)-2-oxobut-3-enoic acid (65 g, 0.309 mol) and 2,4-dichlorophenylhydrazine hydrochloride (65.9 g, 0.309 mol) in glacial acetic acid (780 ml) is heated at 80 ºC using mechanical stirring. At this temperature, the suspension becomes a dark solution. The evolution of the reaction is monitored by HPLC. After 1-2 h, the crude is cooled to 40 ºC and it is carefully dropped over water (780 ml), keeping the temperature between 10-15 ºC. A colored solid is precipitated. The suspension is kept under stirring for at least 30 min. and then it is filtered and washed several times with water (320 ml). The filtrate is resuspended in water (320 ml) and stirred for 1 h to eliminate the acid traces. The solid is filtered and washed again with water until the pH of the mother liquors is over 3. The product is dried in a heat cabinet at 50-60 ºC and a brown solid (108.5 g) containing a 2.19% of water is obtained.

The solid is resuspended in toluene (434 ml) and the mixture was heated at 80 ºC until a solution is obtained. Then, it is cooled to 70-75 ºC, water is added drop by drop and it is cooled to room temperature. The solution is stirred for 1 h and then it is cooled to 0-5 ºC and stirred for one more hour. A white solid precipitates, which is filtered and washed with cold toluene (217 ml) until colorless washing liquors are obtained. The solid obtained is dried in a vacuum heat cabinet at 50 ºC. The hemihydrate is obtained as a white solid (92.2 g, 74 % yield).

IR (KBr, cm⁻¹) : 3200-2200, 1668,4, 1458, 1251,4, 1104,8

¹H RMN (CDCl₃, δ ppm) : 3,3 (dd, 1 H), 3,7 (dd, 1 H), 5,9 (dd, 1 H), 7,09-7,25 (m, 7H).

### c) Preparation of 5-(4-chlorophenyl)-1-(2,4-dichlorophenyl)-N-(piperidin-1-yl)-4,5-dihydro-1H-pyrazole-3-carboxamide (II)

The 5-(4-chlorophenyl)-1-(2,4-dichlorophenyl)-4,5-dihydro-1H-pyrazole-3-carboxylic acid as hemihydrate (45 g, 0.12 moles) is dissolved in toluene (135 ml) and is partially destilled at atmospheric pressure to eliminate water traces. The resulting solution is cooled to 75-85 ºC. DMF is added (2.25 ml) as a catalyst and thionyl chloride (10.4 ml, 0.14 moles) is added drop by drop. The solution is stirred until the complete formation of the acid chloride (monitoring by HPLC or IR). The mixture is cooled to 20-25 ºC and it is added, drop by drop, under nitrogen pressure, over a solution formed by 1-aminopiperidine (15.4 ml, 0.14 mol), toluene (225 ml) and triethylamine (67.5 ml), keeping the temperature of the mixture between 5-10 ºC. A solid precipitates. After the addition, the mixture is warmed to 20-25 ºC and stirred until the reaction finishes (monitoring by HPLC). The suspension formed is treated with water (225 ml) and toluene (90 ml). The mixture is heated to 60-70 ºC until a complete solution is obtained. The organic layer is washed with a 7% NaHCO₃ solution (225 ml) and heated again to 60-70 ºC. Then, the organic layer is washed with a 10% NaCl solution (225 ml) heating to 60-70 ºC again. The final organic extract is partially concentrated under vacuum to eliminate water traces. It is filtered over celite and the solvent is removed completely under vacuum. The residue obtained is dissolved in ethanol (540 ml) and destilled at atmospheric pressure until the final volume is 180 ml. An almost white solid appears. The suspension is cooled to 20-25 ºC and then to 0-5 ºC for 1 h. The solid is filtered and washed with cold ethanol (45 ml).

The solid is resuspended in ethanol (360 ml) and the mixture is heated to reflux and partially destilled (180 ml). A white solid appears. The suspension is cooled to 20-25 ºC, keeping this temperatute for 1 h. Then, it is cooled to 0-5 ºC for 1 more hour and then is filtered. The solid obtained is washed with cold ethanol (45 ml) and is dried in the vacuum heat cabinet at 50 ºC (33.04 g, 62 % yield).

IR (KBr, cm⁻¹) : 3222,9, 2934,9, 1647,4, 1474,7, 1268,3, 815,6.

¹H RMN (CDCl₃, δ ppm) : 1,4 (m, 2H), 1,7 (m, 4H), 2,8 (m, 4H), 3,3 (dd, J=6,1 y 18,3Hz, 1 H), 3,7 (dd, J=12,5 y 18,3 Hz, 1 H), 5,7 (dd, J=6,1 y 12,5 Hz, 1 H), 7,0-7,2 (m, 6H), 7,4 (s, 1 H).

If the substituted pyrazoline compounds of general formula (II) themselves are obtained in form of a mixture of stereoisomers, particularly enantiomers or diastereomers, said mixtures may be separated by standard procedures known to those skilled in the art, e.g. chromatographic methods or fractionalized crystallization with chiral reagents. It is also possible to obtain pure stereoisomers via stereoselective synthesis.

## Claims

1. A process for obtaining the compound 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxylic acid (IIIa) 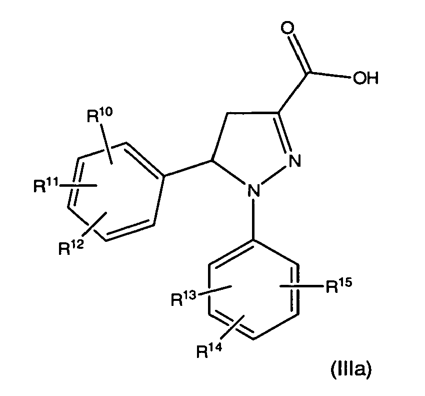 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** at least an intermediate compound of general formula IVb or IVc 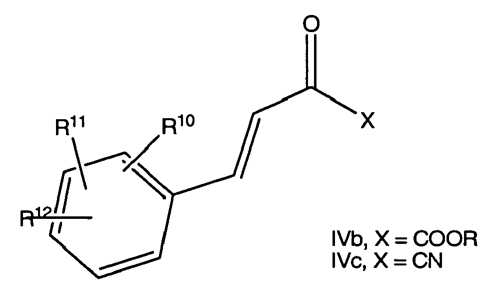 with R being a branched or unbranched C₁₋₆ alkyl radical isolated or generated in situ, reacts with phenylhidrazine (R¹³,R¹⁴,R¹⁵ phenyl substituted) in a suitable reaction medium and suitable reaction conditions.

2. A process for obtaining the compound 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxylic acid (IIIa) 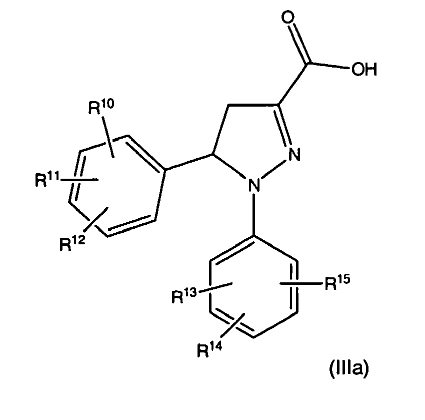 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** at least an intermediate compound of general formula IVa, IVb or lVc 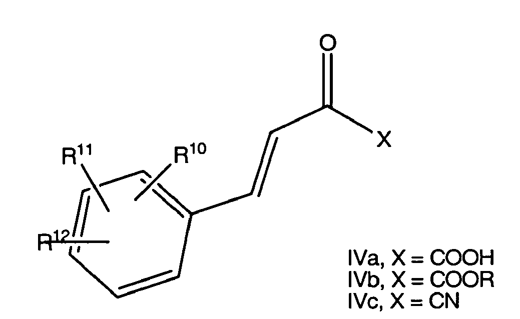 with R being a branched or unbranched C₁₋₆ alkyl radical is an isolated compound with general formula IVa and the reaction with phenylhidrazine (R¹³,R¹⁴,R¹⁵ phenyl substituted) is carried out in C₁₋₄-alcohols or ethers, specially dioxane, tetrahydrofuran or mixtures of at least two of these above mentioned compounds, in the presence of an acid, whereby the acid may be organic such as acetic acid and/or inorganic such as hydrochloric acid, at a temperature from room temperature to the boiling point of the reaction medium and with reaction time from 1 to 2 h.

3. A process for obtaining the compound 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxylic acid (IIIa) 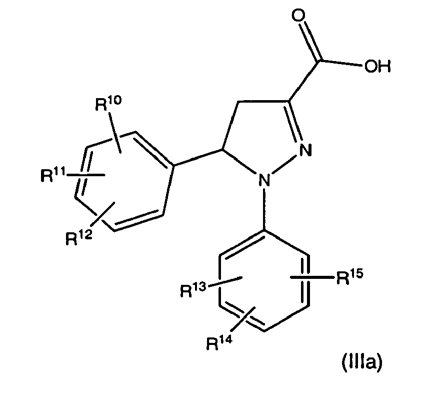 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** comprises the following steps:
a) at least an intermediate compound of general formula IVa, IVb or IVc 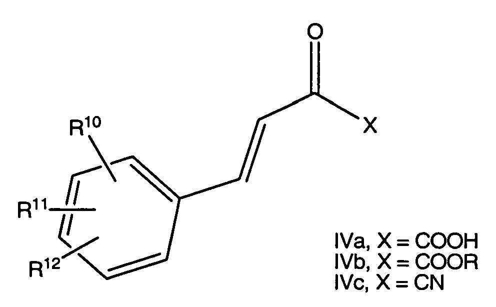 with R being a branched or unbranched C₁₋₆ alkyl radical is derivatized to a phenylthio analog of general formula (VI) 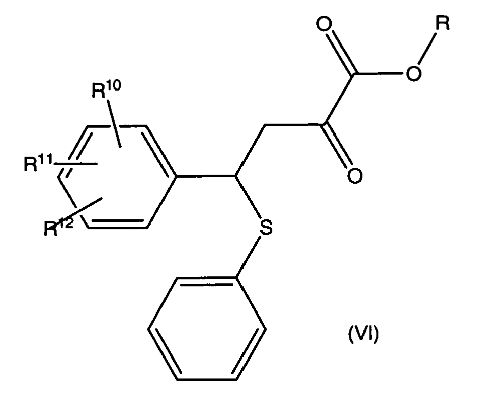 in an aprotic and dry solvent, such as toluene, in the presence of a catalytic amount of an organic base and tiophenol, at a temperature of -20 ºC with reaction time from 4 to 24 hours.
b) the phenylthio analog (VI) reacts in a protic solvent, such as methanol, with a catalytic amount of a base such as KHSO₄ with (R¹³,R¹⁴,R¹⁵)-phenylhydrazine to form the hydrazone of general formula (VII) 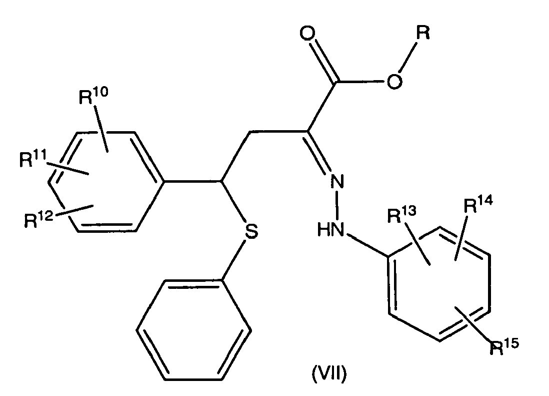 at temperatures from 25 to 50 ºC and with reaction time between 4 to 15 hours.
c) an intramollecular cyclization of the hydrazone (VII) is carried out in an aprotic and dry solvent, such as DMF, under inert atmosphere in the presence of a base, such as NaH.
d) optionally if the starting material is an ester or has an acid equivalent group, such as cyano, instead of the carboxylic group, a saponification or hydroliytic step is needed at the end.

4. A process for obtaining the compound 5-(R¹⁰,R¹¹,R¹²-phenyl)-1-(R¹³,R¹⁴,R¹⁵-phenyl)-N-(piperidin-1-yl)-4,5-dihydro-1H-pyrazole-3-carboxylic acid (IIIa) 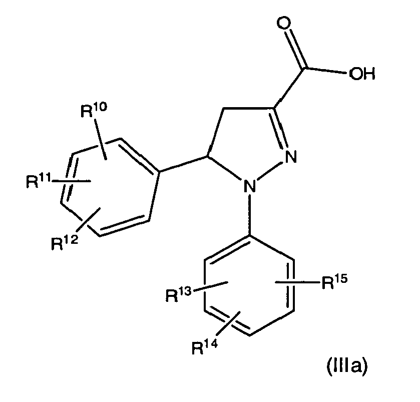 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** comprises the following steps:
a) at least an intermediate compound of general formula IVa, IVb or IVc 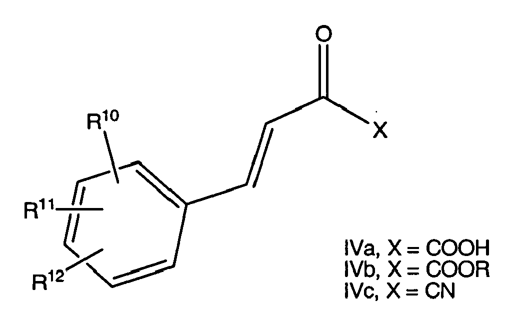 with R being a branched or unbranched C₁₋₆ alkyl radical is brominated to a dibromo derivative of general formula (VIII) 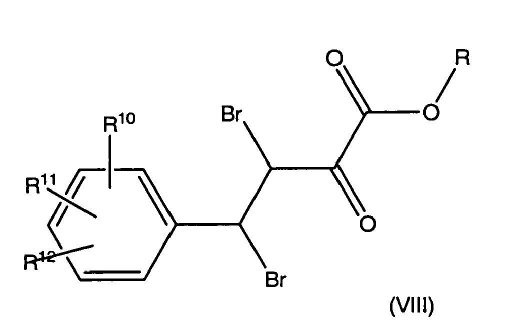 in an aprotic solvent, specially dichloromethane, bromine at room temperature and reaction time from 1 to 2 hours.
b) the dibromo derivative reacts with (R¹³,R¹⁴,R¹⁵)-phenylhydrazine in refuxing DMF from 2 to 3 hours

5. The process according to claims 1 to 4 where a previous step for obtaining a compound of general formula IVa, IVb or IVc is **characterized in that** (R10,R11,R12)-benzaldehyde is condensed with a compound of general formula (IX), (X) or (XI) 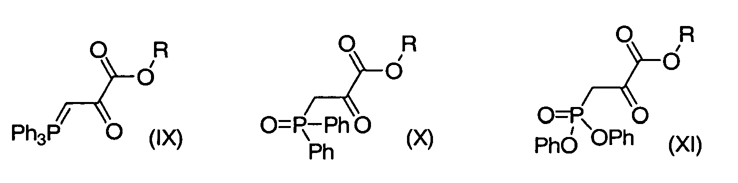 with R being H or a branched or unbranched C₁₋₆ alkyl radical in a Wittig-type reactions conditions as an aprotic solvent, specially tetrahydrofuran, using a base, such as NaH or LDA

6. The process according to claims 1 to 4 where a previous step for obtaining a compound of general formula IVa, IVb or IVc is **characterized in that** (R¹⁰,R¹¹,R¹²)-benzaldehyde is condensed with 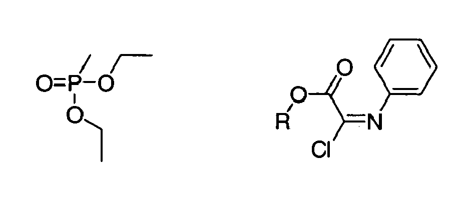 with R being H or a branched or unbranched C₁₋₆ alkyl radical by the following steps:
a) addition of the phosphonate over a base, specially butyllithium, in a dry solvent, specially tetrahydrofuran, at low temperature, preferably -70º C
b) *N*-phenylethoxycarbonylacetimidoyl chloride and (R¹⁰,R¹¹,R¹²)-benzaldehyde are added, the condensation is carried out preferably at room temperature

7. The process according to claim 1 to 4 wherein the intermediate is a compound with general formula IVa, IVb or lVc generated in situ by coupling of (R¹⁰,R¹¹,R¹²)-iodobenzene with a 2-hydroxybut-3-ynoic derivative of general formula (XX) 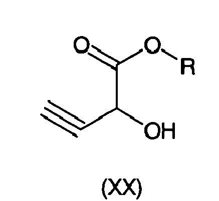 with R being H or a branched or unbranched C₁₋₆ alkyl radical and the reaction is carried out in an aprotic and dry solvent, such as tetrahydrofuran, catalyzed by Pd and Cu, in the presence of a base, such as triethylamine, at a temperature of boiling point of the solvent, adding the phenylhydrazine (R¹³,R¹⁴,R¹⁵ substituted) in a second step after 10-15 hours and being kept heating at reflux from 5 to 10 hours.

8. A process for obtaining 5-((R¹⁰,R¹¹,R¹²)-phenyl)-1-((R¹³,R¹⁴,R¹⁵)-phenyl)-N-(piperidin-1-yl)-4,5-dihydro-1H-pyrazole-3-carboxylic acid (IIIa) 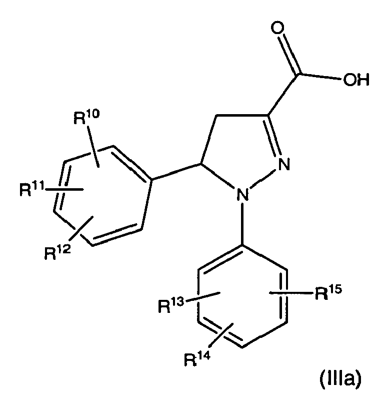 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** comprises the following steps:
a) a compound of general formula (XIII) 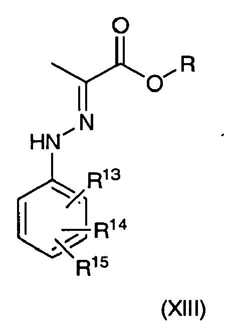 with R being H or a branched or unbranched C₁₋₆ alkyl radical is α-alkylated with (R¹⁰,R¹¹,R¹²)-benzaldehyde in boiling ethanol in the presence of sodium acetate or at room temperature in the presence of glacial acetic acid to form an hydrazone derivative of general formula (XII) 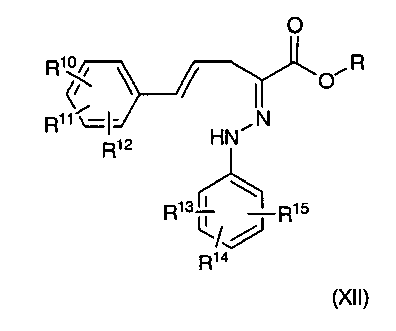
b) an intramollecular cyclization of the hydrazone (XII) is carried out in in boiling ethanol or in boiling water.
c) optionally if the starting material is an ester or has an acid equivalent group, such as cyano, instead of the carboxylic group, a saponification or hydroliytic step is needed at the end.

9. A process for obtaining 5-((R¹⁰,R¹¹,R¹²)-phenyl)-1-((R¹³,R¹⁴,R¹⁵)-phenyl)-N-(piperidin-1-yl)-4,5-dihydro-1H-pyrazole-3-carboxylic acid (IIIa) 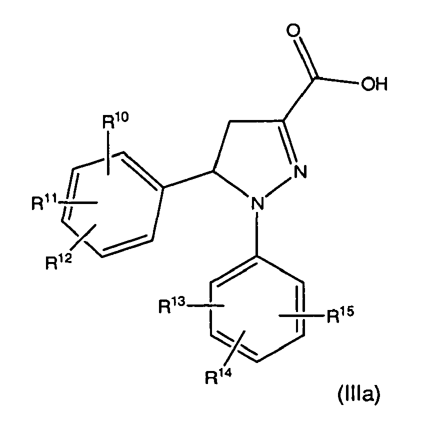 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** at least an intermediate with general formula XIV 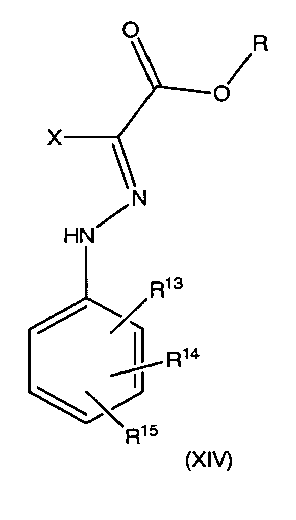 with X being Cl or Br and with R being H or a branched or unbranched C₁₋₆ alkyl radical has a cycloaddition, trough its nitrilimine derived from, with vinylbenzene (R¹⁰,R¹¹,R¹² phenyl substituted) in an aprotic solvent, such as toluene or chloroform or in a protic solvent, such as ethanol, a base, such as triethylamine at temperature between room temperature and 120 º C.

10. The process according to claim 9 wherein the intermediate with general formula (XIV) is generated by the following steps:
a) reaction of an ester of 2-oxoacetic acid and phenylhydrazine (R¹³,R¹⁴,R¹⁵ phenyl substituted) to form an ester of the hydrazone 2-(2-((R¹³,R¹⁴,R¹⁵)-phenyl)hydrazono)acetic in refluxing ethanol and in the presence of some acetic acid and water
b) halogenation of the ester of the hydrazone 2-(2-(-((R¹³,R¹⁴,R¹⁵)-phenyl)hydrazono)acetic acid with a halogenating agent, such as NCS or NBS in an appropriate solvent, such as DMF at room temperature.

11. The process according to claim 9 wherein the intermediate with general formula (XIV) is generated by the following steps:
a) generation of the diazonium salt of (R¹³,R¹⁴,R¹⁵⁾-aniline by the addition of a solution of sodium nitrite in water over the (R¹³,R¹⁴,R¹⁵⁾-aniline in hydrochloric acid
b) reaction of an ester of of 3-oxobutanoic acid and the diazonium salt of (R¹³,R¹⁴,R¹⁵)-aniline in presence of sodium acetate, preferably at pH=4, at room temperature to form an ester of of 2-(2-((R¹³,R¹⁴,R¹⁵)-phenyl)hydrazono)-3-oxobutanoic acid
c) bromination of an ester of 2-(2-((R¹³,R¹⁴,R¹⁵)-phenyl)hydrazono)-3-oxobutanoic acid in the presence of acetic acid, bromine, preferably at room temperature

12. The process according to claim 9 wherein the intermediate with general formula (XIV) is generated by the following steps:
a) generation of the diazonium salt of (R¹³,R¹⁴,R¹⁵⁾-aniline by the addition of a solution of sodium nitrite in water over the (R¹³,R¹⁴,R¹⁵⁾-aniline in hydrochloric acid
b) generation of the dimethylsulfonium bromide derivative by refluxing 2-bromoacetic acid derivative and dimethylsulfide in ethanol for about 30 min
c) reaction of dimethylsulfonium bromide derivative, diazonium salt of (R¹³,R¹⁴,R¹⁵⁾-aniline in the presence of sodium acetate and acetic acid at preferred temperatures from 0 ºC to room temperature.

13. A process for obtaining 5-((R¹⁰,R¹¹,R¹²)-phenyl)-1-((R¹³,R¹⁴,R¹⁵)-phenyl)-N-(piperidin-1-yl)-4,5-dihydro-1H-pyrazole-3-carboxylic acid (IIIa) 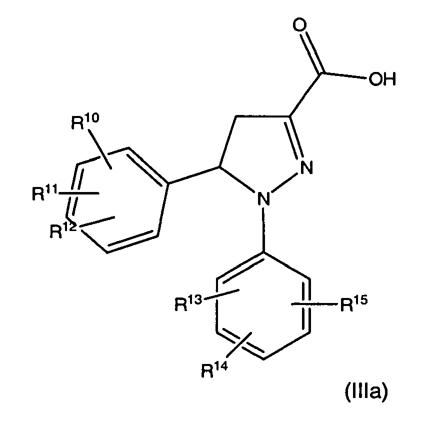 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** a thermolysis of an intermediate of general formula (XV) 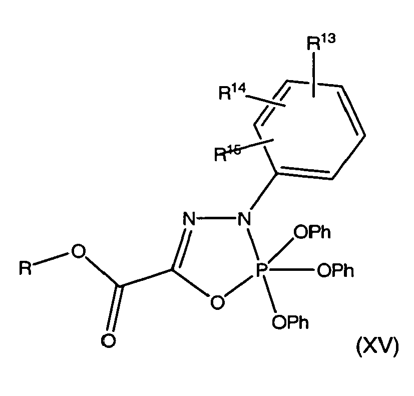 with R being H or a branched or unbranched C₁₋₆ alkyl radical is carried out in the presence of estyrene (R¹⁰,R¹¹,R¹² substituted), refluxing xylene and reaction time from 12 to 24 hours.

14. The process according to claim 13 wherein the phosphole intermediate with general formula (XV) is generated by the following steps:
a) condensation of diethyl oxalate and phenylhydrazine (R¹³,R¹⁴,R¹⁵ phenyl subtituted) to form an ester of the hydrazide 2-(2-((R¹³,R¹⁴,R¹⁵)-phenyl)hydrazinyl)-2-oxoacetic acid (XVI)
b) by the reaction of an ester of the hydrazide 2-(2-((R¹³,R¹⁴,R¹⁵)-phenyl)hydrazinyl)-2-oxoacetic acid (XVI) with phosphorous pentachloride in toluene at room temperature, followed by the addition of a phenol, such as *O*-trimethylsilyl-p-cresol in refluxing toluene.

15. A process for obtaining 5-((R¹⁰,R¹¹,R¹²)-pheny))-1-((R¹³,R¹⁴,R¹⁵)-phenyl)-N-(piperidin-1-yl)-4,5-dihydro-1 H-pyrazole-3-carboxylic acid (IIIa) 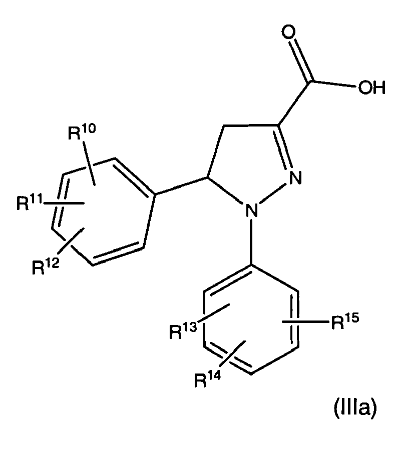 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** comprises the following steps:
a) epoxidation of an intermediate of general formula IVc 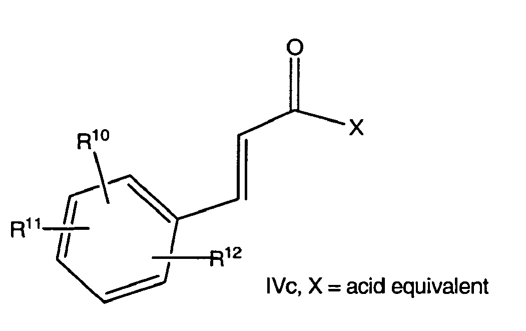 In a suitable reaction media with an oxidant agent, such as perbenzoic acids, sodium peroxocarbonate, hydrogen peroxide, dioxiranes or hydroperoxides
b) sequential nucleophilic attac of phenylhydrazine (R¹³,R¹⁴,R¹⁵ phenyl substituted) to the carbonyl and to the epoxide of the epoxidated intermediate; condensation with the hydrazine is carried out in refluxing ethanol to give an intermediate of general formula (XVII) 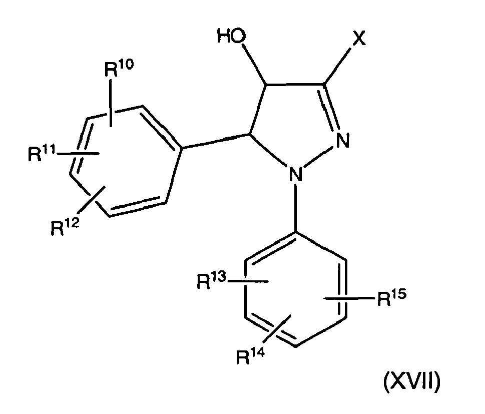 radical deoxygenation of the secondary alcohol (XVII) can be carried out by, for example, a Barton-McCombie reaction using xantates or thiocarbonates and hydrides.

16. A process for obtaining 5-((R¹⁰,R¹¹,R¹²)-phenyl)-1-((R¹³,R¹⁴,R¹⁵)-phenyl)-N-(piperidin-1-yl)-4,5-dihydro-1H-pyrazole-3-carboxylic acid (Illa) 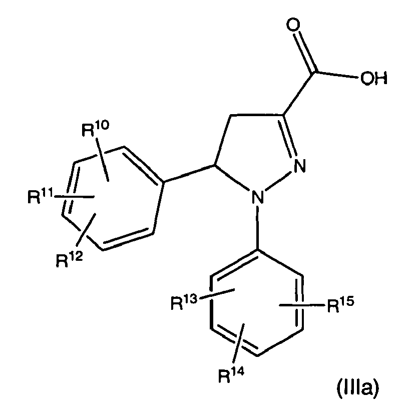 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** comprises the following steps:
a) reaction of phenylhydrazine (R¹³,R¹⁴,R¹⁵phenyl subtituted), (R¹⁰,R¹¹,R¹²)-benzaldehyde and a derivative of 2-oxopropanoic acid to give a derivative of 4-((R¹⁰,R¹¹,R¹²)-phenyl)-4-((R¹³,R¹⁴,R¹⁵)-phenylamino)-2-oxobutanoic acid of general formula (XVIII) 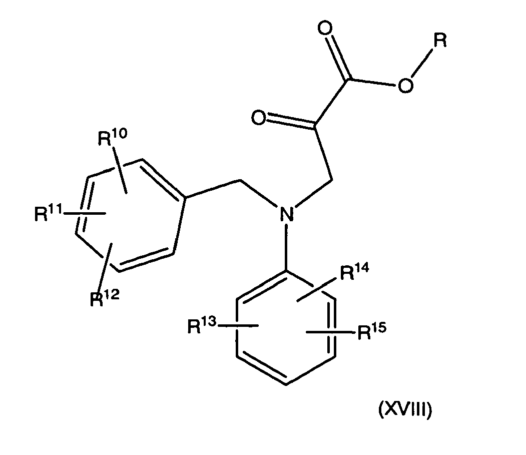 with R being H or a branched or unbranched C₁₋₆ alkyl radical
b) electrophilic amination of the intermediate with general formula (XVIII) carried out following the methodology of Friestad with a variety of *O*-substituted hydroxylamines or by nitrosation using a variety of nitrites, followed by a reduction.
c) intramolecular cyclization wherein trough attack of the primary amine to the carbonyl gives the precursor of the desired acid (IIIa).

17. A process for obtaining a compound of general formula (Ia) 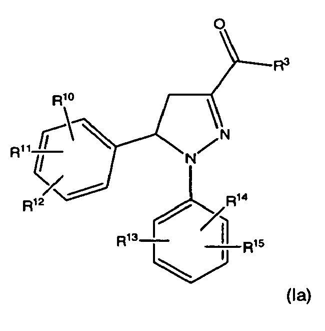 wherein
R³ represents a saturated or unsaturated, optionally at least mono-substituted, optionally at least one heteroatom as ring member containing cycloaliphatic group, which may be condensed with an optionally at least mono-substituted mono- or polycyclic ring system, or R³ represents an optionally at least mono-substituted aryl or heteroaryl group, which may be condensed with an optionally at least mono-substituted mono- or polycyclic ring system, or R³ represents an ―NR⁴R⁵-moiety,
R⁴ and R⁵, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an-SO₂-R⁶-moiety, or an - NR⁷R⁸-moiety,
R⁶ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,
R⁷ and R⁸, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl,
R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl,
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.
**characterized in that** comprises the following steps:
a) Following the production of a compound of general formula (IIIa) according with claims 1 to 16 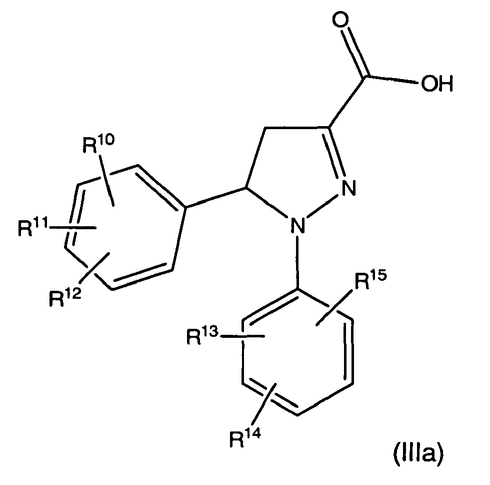 the compound of general formula (IIIa) is transformed to an activated carbonyl derivative
b) the activated carbonyl compound derived from 5-((R¹⁰,R¹¹,R¹²)-phenyl)-1-((R¹³,R¹⁴,R¹⁵)-phenyl)-N-(piperidin-1-yl)-4,5-dihydro-1 H-pyrazole-3-carboxylic acid (IIIa) reacts with an R³H compound, preferable an HNR⁴R⁵, in the presence or absence of an acid receptor or a suitable condensing agent in a suitable reaction media.

18. The process according to claim 17 wherein the activated carbonyl compound derived from 5-((R¹⁰,R¹¹,R¹²)-phenyl)-1-((R¹³,R¹⁴,R¹⁵)-phenyl)-N-(piperidin-1-yl)-4,5-dihydro-1 H-pyrazole-3-carboxylic acid (Illa) is selected from the corresponding acid chloride, an acid anhydride, a mixed anhydride, a straight or branched C₁₋₄ alkyl ester or an activated ester.

19. The process according to claim 17 and 18 wherein the activated carbonyl compound derived from 5-((R¹⁰,R¹¹,R¹²)-phenyl)-1-((R¹³,R¹⁴,R¹⁵)-phenyl)-N-(piperidin-1-yl)-4,5-dihydro-1H-pyrazole-3-carboxylic acid (IIIa) is the acid chloride obtained by chlorination with thionyl chloride, the acid acceptor is an amine base selected from N,N-diisopropylethylamine or triethylamine, the reaction solvent is toluene and the reaction is carried out at temperatures from 70 ºC to 90ºC.

20. The process according to claim 17 wherein the activated carbonyl compound derived from 5-((R¹⁰,R¹¹,R¹²)-phenyl)-1-((R¹³,R¹⁴,R¹⁵)-phenyl)-N-(piperidin-1-yl)-4,5-dihydro-1H-pyrazole-3-carboxylic acid (IIIa) is achieved through activation with carbodiimides.

21. The process according to claim 17 and 20 wherein the carbodiimide is selected from N,N-dicyclohexylcarbodiimide, 1-ethyl-3-(3-dimethylaminopropyl)-carbodiimide (EDC), carbonyldiimadazole, O-benzotriazole-N,N,N',N'-tetramethyl-uronium-hexafluoro-phosphate (HBTU) or benzotriazol-N-oxotris(dimethylamino) phosphonium hexafluorophosphate (BOP)).

22. The process according to claims 17, 20 and 21 wherein the reaction is carried out with 1-ethyl-3-(3-dimethylaminopropyl)-carbodiimide (EDC), 1-hydroxybenzotriazole, inert atmosphere, an aprotic solvent selected from N,N-dimethylformamide or tetrahydrofuran, in presence of a base, especially triethylamine, at room temperature with a time reaction from 15 to 24 hours; use of a polymer-supported EDC (P-EDC) is also a suitable for this condensation

23. The process according to claims 17, 20 and 21 wherein the reaction is carried out with O-Benzotriazole-N,N,N',N'-tetramethyl-uronium-hexafluoro-phosphate (HBTU) as condensating agent, in presence of a base, specially DIPEA, an aprotic solvent, especially acetonitrile, at room temperature with a time reaction from 15 to 24 hours.

24. The process according to claim 17 and 18 wherein the activated carbonyl compound derived from 5-((R¹⁰,R¹¹,R¹²)-phenyl)-1-((R¹³,R¹⁴,R¹⁵)-phenyl)-N-(piperidin-1-yl)-4,5-dihydro-1H-pyrazole-3-carboxylic acid (IIIa) is the mixed anhydride obtained by reaction of (IIIa) with ethylchloroformate and triethylamine, the acid acceptor is an amine base selected from N,N-diisopropylethylamine or triethylamine, the reaction solvent is dichloromethane, inert atmosphere, and the reaction is carried out at temperatures from 0 ºC to room temperature.

25. The process according to claims 17 to 24 for obtaining a compound of general formula (II) 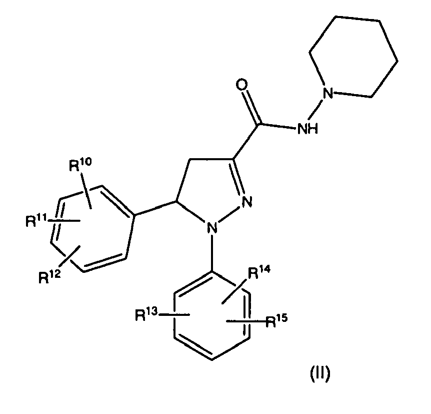 wherein
R¹⁰, R¹¹ and R¹² independently of each other represent hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R¹⁶, SH, SR¹⁶, SOR¹⁶, SO₂R¹⁶, NH₂, NHR¹⁶, NR¹⁶R¹⁷, -(C=O)-NH₂, -(C=O)-NHR¹⁶ or -(C=O)-NR¹⁶R¹⁷ whereby R¹⁶ and R¹⁷ for each substituent independently represent linear or branched C₁₋₆ alkyl, R¹⁴ and R¹⁵ independently of each other represent a linear or branched C₁₋₆-alkyl group, a linear or branched C₁₋₆-alkoxy group, a halogen atom, CH₂F, CHF₂, CF₃, CN, OH, NO₂, -(C=O)-R¹⁸, SH, SR¹⁸, SOR¹⁸, NH₂, NHR¹⁸, NR¹⁸R¹⁹, -(C=O)-NH₂, -(C=O)-NHR¹⁸ and -(C=O)-NR¹⁸R¹⁹, whereby R¹⁸ and optionally R¹⁹ for each substituent independently represent linear or branched C₁₋₆ alkyl;
R¹³ represents hydrogen, a linear or branched C₁₋₆-alkyl group, a linear or branched C₁₋₆-alkoxy group, a halogen atom, CH₂F, CHF₂, CF₃, CN, OH, NO₂, -(C=O)-R²⁰, SH, SR²⁰, SOR²⁰, NH₂, NHR²⁰, NR²⁰R²¹, -(C=O)-NH₂, -(C=O)-NHR²⁰ and -(C=O)-NR²⁰R²¹, whereby R²⁰ and optionally R²¹ for each substituent independently represent linear or branched C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

26. The process according to claims 17 to 24 for obtaining N-piperidin-1-yl-5-(4-chloro-phenyl)-1-(2,4-dichlorophenyl)-4,5-dihydro-1 H-pyrazole-3-carboxamide
